# EUROPEAN PATENT APPLICATION

(11) **EP 1 314 716 A1**
(43) Date of publication of application: **28.05.2003**
(21) Application number: 01204522.5
(22) Date of filing: 23.11.2001
(51) Int. Cl.: C07C 215/48, C07C 215/52, C07C 229/36, C08G 59/62

(54) **Amine phenolic compounds and their use as hardener and/or accelerators in epoxy coatings**

(71) Applicant: SIGMA COATINGS B.V., 1422 AD Uithoorn (NL)
(72) Inventor: Mill, Sibel, 1470 Bousval (BE); Gerritsen, Roy, 1326 LB Almere (NL); De Vries, Gerard, 1078 AS Amsterdam (NL)
(74) Representative: Walsh, David Patrick

(57) **Abstract**

The invention relates to new having the formula HO-C₆H₅₋ₙ-(XN(Y¹N(R¹)R²)Y²N(R³)R⁴)ₙ wherein n is an integer chosen from 1 to 3; X, Y¹, Y² represent each independently a bivalent radical and R¹, R², R³ and R⁴ represent each independently a hydrogen, alkyl or aminoalkyl in which at least one of R¹, R², R³,R⁴ or Y¹N(R¹)R² represent hydrogen. A method for the preparation of the new compounds is described, which comprises reacting an aminophenolic compound with a suitable reagent and reducing the intermediate.

## Description

### Field of the invention

The invention relates to new amine phenolic compounds, their method of preparation and their use as hardener and/or accelerators in coatings.

### Background of the invention

One of the main components of epoxy resin systems, beside the epoxy resin, is the curing agent, also know as hardener. Amine compounds are the most widely used curing agents or hardeners in epoxy resin systems in the industrial and protective coatings business to coat and protect metallic surfaces. These amine curing agents may be aromatic, as well as aliphatic. Other known curing agents include polyamides, which are adducts of dimeric fatty acid with diprimary amines, amidoamines which are adduct of monomeric fatty acid with diprimary amines, phenalkamines which are derived from n-pentadecenylphenol, a natural constituent of cashew nutshell oil and Mannich bases (Protective coatings: Fundamentals of chemistry and Composition, Clive H. Hare, Technology Publishing Company, Pittsburgh, 1994 and Weinmann, D.J., Dangayach, K. Smith C., J. Coat. Technol. 1996, 68 (863), 29-37). Mannich bases are aminomethylated products produced by a Mannich reaction, which is the aminomethylation of carbonyl compounds and other acidic CH compounds by the aldol condensation of active methylene groups with non-enolizable aldehydes and ammonia or primary or secondary amines. Mannich bases useful as curing agents, are formed from phenol, formaldehyde and amines.

Curable mixtures based on epoxy resins and on known amine compounds may have long curing times at room temperature or below. One known technology for speeding up the drying time and for increasing the cure rate of epoxy/amine coatings for low temperature application is to accelerate the epoxy/amine reaction, using accelerators. Several compounds are known to accelerate this cross-linking reaction, such as tertiary amines, organic acids like salicylic and benzoic acid, phenols, alcohols, water (Weinmann, D.J., Dangayach, K. Smith C., J. Coat. Technol. 1996, 68 (863), 29-37 and 'Handbook of epoxy resins", Lee & Neville, 1967, chap. 10, table 10-9). However these compounds are not chemically incorporated in the polymeric coating and thus can be leached out of the coating resulting in embrittlement. These compounds are sometimes also referred to as catalysts: these compounds are usually small and polar, so they attract water into the film, making it water-sensitive. Shorter pot life, is another disadvantage of accelerated epoxy coatings systems, which results in a rapid increase in viscosity affecting the applicability. The time during which the coating composition is low enough in viscosity to be applied to the substrate is much shorter.

US Pat. No 4,269,742 relates to amine compounds, adaptable to use as a hardener for epoxy compounds, which are the reaction products of Mannich bases (prepared from phenols, secondary monoamines and formaldehyde) with aminoamides, aminoimidazolines, imidazoline containing aminoamides, and /or polyamines, with amine exchange and the splitting-off of secondary amine.

WO 00/15687 relates to accelerators for hardenable epoxy and polyurethane systems. These compounds are the result of a transamination reaction between a substituted phenolic base and a polyamine.

It is a main objective to provide new compounds, suitable as hardeners and/or accelerators in coatings systems such as epoxy systems. It is another object of the invention to provide new compounds, which can be incorporated in the epoxy-amine network providing better physical properties to the coatings, such as faster cure at reduced temperatures, shorter recoating intervals leading to increased productivity, insensibility to water, better film hardness development, etc. It is another objective to provide new compounds suitable as hardeners and/or accelerators, which permit the increase of the curing rate of coatings. It is a further objective of the invention to provide new compounds that permit the fast curing of coating systems at room temperature or below. Yet a further objective is to provide a quick and easy method for the preparation of said new compounds.

### Summary of the invention

The invention relates to new compounds having the general formula (I), 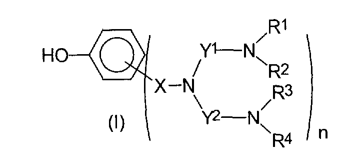 wherein n is an integer chosen from 1 to 3, X, Y¹, Y² represent each independently a bivalent radical, and R¹, R², R³, R⁴ represent each independently a hydrogen, alkyl or aminoalkyl in which at least one of R¹, R², R³, R⁴ represents hydrogen. According to an embodiment the present invention relates to compounds of formula (I) wherein Y¹-N(R¹)R² is H. According to another embodiment said compound of formula (I) comprises at least one primary amine.

These compounds have shown to have surprisingly good properties as hardeners. These compounds can also be used as accelerators in coating systems such as epoxy systems. These compounds have a good reactivity as hardener as they contain at least one primary amine functional moiety providing a better cross-linking reaction in the coating system. Furthermore, the presence of the phenol group in said compounds produces substantial accelerating action on the epoxy-amine reaction, by enhancing the rate of nucleophilic ring opening of the epoxide by the amine curing agent.

The present invention will be further disclosed in detail hereunder. Examples are given which will further support the description.

### Detailed description

The present invention relates to new compounds having the above-described formula (I), which are suitable as hardener and/or accelerators in coating systems such as epoxy systems.

The term "accelerator" as used herein relates to a compound that accelerates the overall rate of the reaction between a base resin such as an epoxy and a hardener such as an amine. The accelerator is added as a small quantity (a few percent in equivalent to the epoxy) in presence of the amine hardener (which is present in stoichiometric equivalent to the epoxy). So the accelerator can be a catalyst (not chemically incorporated in the film), but also a molecule which acts as a catalyst and reacts with the epoxy (and is chemically incorporated into the polymeric film). The advantage of the last case is that the use of a conventional amine is still possible.

According to an embodiment, the present invention relates to a compound of formula (I) wherein X is selected from the group consisting of alkyl, alkoxy, aralkyl, thio derivatives optionally substituted by alkyl, aryl, cycloalkyl, halogen, hydroxy, oxy derivatives, thiol, thio derivatives, amino, amino derivatives, amido, amidoxy, nitro, cyano, keto, acyl derivatives, acyloxy derivatives, carboxy, ester, ether, esteroxy, sulfonic acid, sulfonyl derivatives, sulfinyl derivatives, heterocycle, alkenyl or alkynyl, and Y¹, Y² each independently are selected from the group consisting of alkyl, alkoxy, aralkyl, optionally substituted by alkyl, aryl, cycloalkyl, halogen, hydroxy, oxy derivatives, thiol, thio derivatives, amino, amino derivatives, amido, amidoxy, nitro, cyano, keto, acyl derivatives, acyloxy derivatives, carboxy, ester, ether, esteroxy, sulfonic acid, sulfonyl derivatives, sulfinyl derivatives, heterocycle, alkenyl or alkynyl.

The term "alkyl", as used herein, includes saturated monovalent hydrocarbon radicals having straight, branched or cyclic moieties or combinations thereof and contains 1-20 carbon atoms, preferably 1-6 carbon atoms. Preferred alkyl radicals are methyl, ethyl, propyl, isopropyl, butyl, iso- or tertiobutyl and pentyl. Preferred substituted alkyl radical are methyl, ethyl and propyl, substituted by at least a group selected from alkyl, hydroxyl, carbonyl and carboxyl. The term "alkoxy" as used herein, relates to -O-alkyl, wherein alkyl is as described above. The term "aralkyl" as used herein, relates to a group of the formula alkyl-aryl in which alkyl is as defined above. The term "aryl" as used herein, includes an organic radical derived from an aromatic hydrocarbon by removal of one hydrogen, such as phenyl, biphenyl, naphthyl, which can optionally be substituted with any suitable group, including but not limited to one or more moieties as described above for the alkyl groups.

The term "halogen", as used herein, includes an atom of Cl, Br, F, I. The term "hydroxy", as used herein, represents a group of the formula -OH. The term "oxy derivatives", as used herein includes -O-R¹⁰ groups wherein R¹⁰ is defined below. Non-limiting examples are methoxy, ethoxy, pentyloxy, propyloxy, phenoxy, benzyloxy, R¹⁰ and R¹¹ are independently selected from the group consisting of alkyl, alkenyl, alkynyl, cycloalkyl, cycloalkenyl, heterocycle, aryl and aralkyl.

The term "heterocycle", as used herein, refers to an aromatic or non aromatic cyclic alkyl, alkenyl, or alkynyl moiety as defined above, having at least one O, S and/or N atom interrupting the carbocyclic ring structure and optionally, one of the carbon of the carbocyclic ring structure may be replaced by a carbonyl.

The term "thiol", as used herein, represents a group of the formula -SH. The term "thio derivatives" as used herein includes -S-R¹⁰ or R¹⁰-S-R¹¹ or R¹⁰-S- groups wherein R¹⁰ and R¹¹ are defined above.

The term "amino", as used herein, represents a group of the formula -NH₂. The term "amino derivatives" as used herein, includes -NHR¹⁰ or -NR¹⁰R¹¹ groups wherein R¹⁰ and R¹¹ are defined above. The term "aminoalkyl" as used herein, includes -NHR¹⁰ or -NR¹⁰R¹¹ groups wherein R¹⁰ and R¹¹ are alkyl. The term "amido" means a group of formula -CONH₂, -CONHR¹⁰ or -CONR¹⁰R¹¹ wherein R¹⁰ and R¹¹ are defined above. The term "amidooxy" means a group of formula -O-CONH₂ or -O-CONHR¹⁰ or -O-CONR¹⁰R¹¹ wherein R¹⁰ and R¹¹ are defined above.

The term "nitro", as used herein, represents a group of the formula -NO₂. The term "cyano", as used herein, represents a group of the formula -CN. The term "keto", as used herein, represents a group of the formula C=O. The term "acyl derivative" as used herein, represents a radical derived from carboxylic acid and thus includes groups of the formula R¹⁰-CO-, wherein R¹⁰ is defined above and may also be hydrogen. Non-limiting examples are formyl, acetyl and propionyl. The term "acyloxy derivatives" as used herein, represents a radical of carboxylic acid and thus includes groups of the formula R¹⁰-CO-O-, wherein R¹⁰ is defined above and may also be hydrogen. The term "carboxy", as used herein, represents a group of the formula -COOH. The term "ester" means a group of formula -COO-R¹⁰ wherein R¹⁰ is defined above.

The term "ether" means a group selected from straight or branched alkyl, alkenyl or alkynyl groups or a combination of the same, interrupted by one or more oxygen atoms. The term "esteroxy" means a group of formula -O-COO-R¹⁰, wherein R¹⁰ is defined above and may also be hydrogen.

The term "sulfonic acid", as used herein, represents a group of the formula -SO₃H. The term "sulfonyl derivative" represents a group of the formula -SO₂-R¹⁰, wherein R¹⁰ is defined above. The term "sulfinyl derivative" represents a group of the formula -SO-R¹⁰, wherein R¹⁰ is defined above.

The term "alkenyl" refers to a straight or branched hydrocarbon with at least one double bond, optionally substituted with any suitable group, including but not limited to one or more moieties as described above for the alkyl groups. Non limiting examples are vinyl, propenyl, butenyl, pentenyl, hexenyl, decenyl, isopropenyl, and isopentenyl. The term "alkynyl" refers to a straight or branched hydrocarbon with at least one triple bond, optionally substituted with any suitable group, including but not limited to one or more moieties as described above for the alkyl groups. Non-limiting examples are ethynyl and propynyl.

According to another embodiment the present invention relates to a compound as defined above, wherein X, Y¹, Y² represent each independently a C₁₋₆alkyl.

According to another embodiment, the present invention relates to a compound of formula (I) wherein R¹, R², R³, R⁴ represent each independently a hydrogen or a C₁₋₆ alkyl or C₁₋₆ aminoalkyl.

More preferably, the present invention relates to a compound of formula (I) wherein X, Y¹, Y² each independently are selected from the group consisting of ethyl, propyl, butyl and pentyl.

According to another embodiment, the present invention relates to a compound as defined above wherein at least two of R¹, R², R³, R⁴ are hydrogen, preferably at least three and most preferably four of R¹, R², R³, R⁴ are hydrogen.

According to another embodiment, the invention relates to compound of the general formula (I) wherein n is 1, and said compound is being para substituted.

In another embodiment, the present invention relates to a compound as defined above having the formula of N,N-(diaminopropyl)-4-(2-aminoethyl)phenol, N,N-(diaminopropyl)-4-(2-aminoethyl-1-ol)phenol, N,N-(diaminopropyl)-4-hydroxyphenylglycine, N,N-(diaminopropyl)-4-hydroxyphenylglycine methyl ester, N,N-(diaminopropyl)-tyrosine, or N,N-(diaminopropyl)-tyrosine methyl ester.

The invention further relates to a method for the preparation of said compounds, comprising the steps of (a) reacting an amino phenolic compound of formula (II) with a suitable reagent resulting in an intermediate compound of formula (III), and 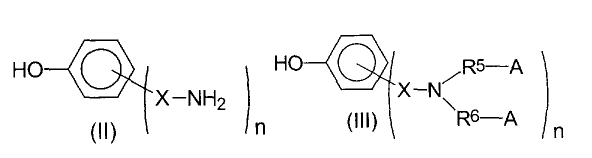
- (b) reducing said compound of formula (III) in order to obtain a compound of formula (I), 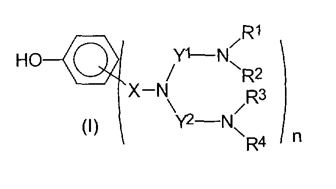
- (c) optionally iterating step (a) and (b) on each compound obtained after step (b),
wherein, R⁵ and R⁶ correspond respectively to Y¹ and Y² with one CH₂ moeity less, A is -C≡N or NO₂ and n is an integer chosen from 1 to 3, X, Y¹, Y² represent each independently a bivalent radical, and R¹, R², R³, R⁴ represent each independently a hydrogen, alkyl or aminoalkyl in which at least one of R¹, R², R³, R⁴ represents hydrogen.

According to an embodiment, R⁵-A is H in intermediate compound of formula (III) and respectively Y¹-N(R¹)R² is H in compound of formula (I). These compounds are the result of the addition of one molecule of suitable reagent on the compounds of formula (II), and not two. A mixture of compound of formula (III) wherein R⁵-A is H and wherein R⁵-A is different from H can be also obtained.

Examples of phenolic compounds of formula (II) containing a primary amine, include but are not limited to -4-(2-aminoethyl)phenol (tyramine), 4-(2-aminoethyl-1-ol)phenol (octopamine), 4-hydroxyphenylglycine, 4-hydroxyphenylglycine methyl ester, tyrosine, tyrosine methyl ester as well as other phenolic derivative containing at least one primary amino group.

According to an embodiment, the reaction of a compound of formula (II) with a suitable reagent forming intermediate compound of formula (III) may be carried out in a solvent suitable for said reaction, such as for example methanol, preferably under reflux conditions.

Suitable reagents are compounds of the formula (IVa) or (IVb), 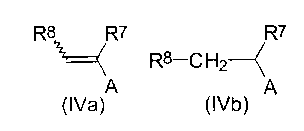 wherein A is -C≡N or NO₂, R⁷ is H or CH₃, R⁸ is H, CH₃, a hydrocarbon radical having 2-18 carbon atoms and containing at least one double bond optionally conjugated with the double bond of formula (IVa) or -R⁹-A wherein R⁹ is a C₁₋₆ alkyl or C₂₋₆ alkenyl radical containing at least one double bond optionally conjugated with the double bond of formula (IVa) and A has the same meaning as that defined above. Examples of suitable reagent include but are not limited to methacrylonitrile and acrylonitrile.

According to another embodiment the present invention further relates to intermediate compounds of formula (III) obtainable by a method according to the invention. In a preferred embodiment, the present invention relates to intermediates having the formula of N,N-(dicyanopropyl)-4-(2-aminoethyl) phenol, N,N-(dicyanopropyl)-4-(2-aminoethyl-1-ol) phenol, N,N-(dicyanopropyl)-4-hydroxy phenylglycine, N,N-(dicyanopropyl)-4-hydroxy phenylglycine methyl ester, N,N-(dicyanopropyl)-tyrosine, or N,N-(dicyanopropyl)-tyrosine methyl ester.

The reduction of intermediate compound of formula (III) to compound of formula (I) may be carried out using different methods known in the art. Non limiting examples of reduction reactions are catalytic hydrogenation, reduction with complex metal hydrides (such as lithium aluminum hydride).

According to another embodiment, the reduction of intermediate compound of formula (III) to compound of formula (I) may be done by catalytic hydrogenation. The reaction can be optionally carried out in a solvent suitable for catalytic hydrogenation. Examples of catalyst useful for said reduction include but is not limited to platinum (IV) oxide, palladium oxide, Raney nickel, Raney cobalt, copper chromite, rhodium oxide, iridium, ruthenium. In a preferred embodiment, the catalyst used in said reduction is platinum (IV) oxide or Raney Cobalt.

In the method of preparation of a compound according to the invention, said compound may be isolated from the reaction medium and, if necessary, further purified according to methodologies generally known in the art such as, for example extraction, crystallization, distillation, trituration and chromatography, or any combination of the same.

The present invention also encompasses alternative method for the synthesis of the compounds according to the invention. Said method comprises the steps of
(a) reacting compound of formula (II) with aziridine to obtain compound of formula (IV). 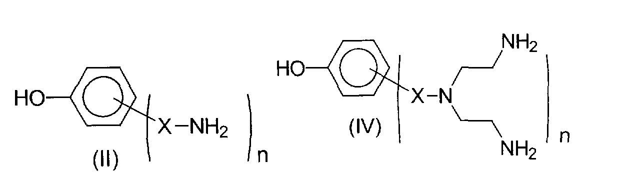
(b) reacting said compound formula (IV) with a compound of formula (IVa) and further performing a catalytic hydrogenation to obtain compound of formula (VII), 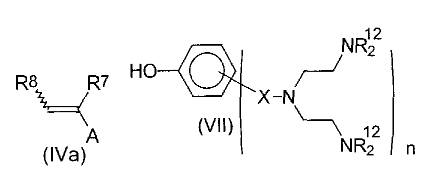
wherein R¹² is H or aminoalkyl, and n, X, R⁷, R⁸ and A have the same meaning as that defined above. Step (a) and/or step (b) can be further iterated.

The present invention further relates to the use of a compound as defined above in coating systems such as epoxy coatings. The present compounds may have multiple functional groups such as at least one or more primary amine groups and a phenol group presenting therefore a plurality of reactive sites. Due to the accelerating effect of the phenolic group the compounds according to the invention are good accelerators of the epoxy-amine reaction, in presence of any conventional amino hardener. The present compounds also showed fast curing times and cured at lower temperatures. Said compounds are therefore useful in providing short curing times of coating systems, especially in systems curable at room temperature or below.

Examples of suitable epoxy resins, are resins which can be produced by the attachment of an epoxide group to both ends of a paraffinic hydrocarbon chain (for example, diepoxides derived from butanediol) or of a polyether chain, such as α-ω-diepoxy polypropylene glycol. More exotic diepoxy resins include but are not limited to vinylcyclo hexene dioxide, 3,4-epoxycyclohexylmethyl 3,4-epoxycyclohexanemono carboxylate, 3-(3,4-epoxycyclohexyl)-8,9-epoxy-2,4-dioxaspiro-[5.5]undecane, bis(2,3-epoxycyclopentyl) ether, bis(3,4-epoxy-6-methylcyclohexyl) adipate and resorcinol diglycidyl ether. Other epoxy resins employed can contain more than two epoxide functional groups per molecule, such as epoxidized soya oils, polyglycidyl ethers of phenolic resins of the novolak type, p-aminophenoltriglycidyl ether or 1,1,2,2-tetra(p-hydroxyphenyl)ethane tetraglycidyl ether. Another class of epoxy resins comprises the epoxy polyethers obtained by reacting an epihalohydrin (such as epichlorohydrin or epibromohydrin) with a polyphenol in the presence of an alkali. Suitable polyphenols include resorcinol, catechol, hydroquinone, bis(4-hydroxyphenyl)-2,2-propane, i.e. bisphenol A; bis(4-hydroxyphenyl)-1,1-isobutane, 4,4-dihydroxybenzophenone; bis(4-hydroxyphenyl-1,1 -ethane; bis(2-hydroxynaphenyl)-methane; and 1,5-hydroxynaphthalene. One very common polyepoxide is a polyglycidyl ether of a polyphenol, such as bisphenol A. Another class of epoxy resin consists of the hydrogenated epoxy resin based on bisphenol A such as Eponex 1510 from Shell. Other classes of epoxy resins are the polyglycidyl ethers of polyhydric alcohols. These compounds may be derived from such polyhydric alcohols as ethylene glycol, diethylene glycol, triethylene glycol, 1,2- propylene glycol, 1,4-butylene glycol, 1,5-pentanediol, 1,2,6-hexane- triol, glycerol, trimethylolpropane, and bis(4-hydroxycyclohexyl)-2,2- propane. A detailed list of suitable epoxide compounds can be found in the handbooks A. M. Paquin, "Epoxidverbindungen und Harze" (Epoxide Compounds and Resins), Springer Verlag, Berlin 1958, Chapter IV and H. Lee and K. Neville, "Handbook of Epoxy Resins" MC Graw Hill Book Company, New York 1982 Reissue, as well as C. A. May, "Epoxy Resins-Chemistry and Technology", Marcel Dekker, Inc. New York and Basle, 1988.

Examples of conventional amine hardener include but are not limited to aliphatic, cycloaliphatic amine, aromatic, araliphatic amines, imidazoline group-containing polyaminoamides based on mono or polybasic acids, as well as adducts thereof. These compounds are part of the general state of the art and are described, inter alia, in Lee & Neville, "Handbook of Epoxy Resins", MC Graw Hill Book Company, 1987, chapter 6-1 to 10-19.

The invention further relates to a curing composition comprising an epoxy resin, and a compound according to the invention, wherein said compound is preferably in an amount ranging from 0 to 70 % by weight when used as hardener and in an amount ranging from 0 to 30 % by weight when used as accelerator. According to another embodiment, the curing composition is a coating composition comprising an epoxy resin, and a compound according to the invention, wherein said compound is preferably in an amountranging from 0 to 70 % by weight when used as hardener and in an amount ranging from 0 to 30 % by weight when used as accelerator.

Suitable epoxy resins are described above. In another embodiment, the invention relates to a curing composition, wherein the epoxy resin is bisphenol A diglycidylether (DGEBA).

If appropriate, the curing composition can be diluted with amines in order to establish optimum curing properties. Useful amines in this regard include polyamines distinguished by the fact that it carries at least two primary amino groups, in each case bonded to an aliphatic carbon atom. It can also contain further secondary or tertiary amino groups. Suitable polyamines include polyaminoamides (from aliphatic diamines and aliphatic or aromatic dicarboxylic acids) and polyiminoalkylene-diamines and polyoxyethylene-polyamines, polyoxypropylene-polyamines and mixed polyoxyethylene/polyoxypropylene-polyamines, or amine adducts, such as amine-epoxy resin adducts. Said amines may contain 2 to 40 carbon atoms. For examples, the amines can be selected from polyoxyalkylene-polyamines and polyiminoalkylene-polyamines having 2 to 4 carbon atoms in the alkylene group, and have a number-average degree of polymerization of 2 to 100, other examples of amines can be linear, branched or cyclic aliphatic primary diaminoalkanes having 2 to 40 carbon atoms. In addition, said amines can be araliphatic amines having at least two primary amino groups, each of which are bonded to an aliphatic carbon atom. The curing composition can include these amines in an amount ranging from 5 to 90% by weight, or for example in an amount ranging from 5 to 40% by weight and for example in an amount ranging from 10 to 40% by weight. Examples of suitable polyamines include: 1,2-diaminoethane, 1,2-diaminopropane, 1,3-diaminopropane, 1,4-diaminobutane and higher homologues, as well as 2-methyl-1,5-diaminopentane, 1,3-diaminopentane, 2,2,4-trimethyl-1,6-diaminohexane and 2,4,4-trimethyl-1,6-diaminohexane as well as industrial mixtures thereof, 1-amino-3-aminomethyl-3,5,5-trimethylcyclohexane, 2,2-dimethyl-1,3-diaminopropane, 1,3-bis(aminomethyl)cyclohexane, 1,2-diaminocyclohexane, 1,3-bis(aminomethyl)benzene, bis(4-aminocyclohexyl)methane, bis(4-amino-3-methylcyclohexyl)methane, 3-azapentane-1,5-diamine, 4-azaheptane-1,7-diamine, 3,6-diazaoctane-1,8-diamine, benzyloxypropylaminepropylamine, diethylaminopropylamine, 3(4),8(9)-bis(aminomethyl)tricyclo-[5.2.1.0^{2,6}]decane, 3-methyl-3-azapentane-1,5-diamine, 3,6-dioxaoctane-1,8-diamine, 3,6,9-trioxaundecane-1,11-diamine, 4,7-dioxadecane-1,10-diamine, 4, 7,10-trioxatridecane-1,13-diamine, 4-aminomethyl-1,8-diaminooctane, 2-butyl-2-ethyl-1,5-diaminopentane, 3-(aminomethyl)benzylamine (MXDA), 5-(aminomethyl)bicyclo[[2.2.1]hept-2-yl]methylamine (NBDA), polyamino imidazoline (Versamid 140), as well as diethylenetriamine (DETA), triethylenetetramine (TETA, which is a mixture of several polyamines), pentaethylenetetramine, dimethyldipropylenetriamine, dimethylaminopropyl-aminopropylamine (DMAPAPA), N-2-(aminoethyl)piperazine (N-AEP), N-(3-aminopropyl)piperazine, norbornane diamine, epilink MX, isophorondiamine (IPD), diaminodicyclohexylmethane (PACM), dimethyldiaminodicyclohexyl methane (Laromin C260), tetramethylhexamethylenediamine (TMD), bis aminomethyl-dicyclopentadiene (tricyclodecyldiamine, TCD), diaminocyclohexane, diethylaminopropylamine (DEAPA), and the like. Suitable polyoxyalkylenepolyamines can be obtained, for example, under the trade name ®Jeffamine such as polyoxypropylene triamine (Jeffamine T403) and polyoxypropylene diamine (Jeffamine D230), and suitable polyiminoalkylenepolyamines are available, for example, under the trade name ®Polymin. Also mixtures from several amines are possible.

Primary aliphatic monoamines can also be added to the curing composition. Suitable monoamines include, for example, unbranched 1-aminoalkanes with for example a saturated alkyl radical of 6 to 22 carbon atoms. The higher representatives of this class of compounds also are called fatty amines. Non-limiting examples include laurylamine, stearylamine, palmitylamine and biphenylamine. However, monoamines with branched chains also are suitable, for example 2-ethylhexan-1-amine or 3,5,5-trimethylhexan-1-amine. amino-2-butane, methoxypropylamine, isopropoxypropylamine. They can be employed individually or as a mixture, and in particular in an amount ranging from 0.1 to 10 %, and for example in an amount ranging from 1 to 5 %.

If appropriate, the curing composition according to the invention may additionally comprise a diluent that is inert. Examples of suitable diluents include aliphatic linear, branched or cyclic ethers having 4 to 20 carbon atoms and mixed aliphatic-aromatic ethers having 7 to 20 carbon atoms, such as dibenzyl ether, tetrahydrofuran, 1,2-dimethoxyethane or methoxybenzene; aliphatic linear, branched or cyclic or mixed aliphatic-aromatic ketones having 4 to 20 carbon atoms, such as butanone, cyclohexanone, methyl isobutyl ketone or acetophenone; aliphatic linear, branched or cyclic or mixed aromatic-aliphatic alcohols having 4 to 20 carbon atoms, such as methanol, ethanol, butanol, 2-propanol, isobutanol, isopropanol. benzyl alcohol, methoxypropanol or furfuryl alcohol; aliphatic linear, branched or cyclic or mixed aromatic-aliphatic esters such as methoxypropylacetate or DBE (dibasic esters from Dupont, mixture of dimethyl adipate, succinate and glutarate); aliphatic linear, branched or cyclic or mixed aromatic-aliphatic hydrocarbons such as toluene, xylene, heptane and mixtures of aliphatic and aromatic hydrocarbons having a boiling range above 100 °C. under normal pressure, as well as low-viscosity coumarone-indene resins or xyleneformaldehyde resins. Aliphatic alcohols having one phenyl radical, such as benzyl alcohol, 1-phenoxypropane-2,3-diol, 3-phenyl-1-propanol, 2-phenoxy-1-ethanol, 1-phenoxy-2-propanol, 2-phenoxy-1-propanol, 2-phenylethanol, 1-phenyl-1-ethanol or 2-phenyl-1-propanol, are preferred. The diluents can be employed individually or as a mixture, and in particular in a amount ranging from 1 to 35 % by weight, for example in an amount ranging from 5 to 25% by weight and for example in an amount ranging from 10 to 30 %.

The curing composition may also contain auxiliaries or additives such as solvents, colorants, mineral oils, fillers, elastomers, antioxidants, stabilizers, defoamers, extenders, plasticizers, catalysts, pigments, pigment pastes, reinforcing agents, flow control agents, thickening agents, flame-retarding agents, additional hardeners and additional curable compounds, depending on the application.

Curing of the composition according to the invention typically proceeds very rapidly, and in general can take place at a temperature within the range of from -10 °C to +50 °C, in particular from 0 °C to 40 °C, more in particular from 3 °C to 20 °C.

The curing compositions according to the invention can find various industrial applications because of their favorable properties such as fast curing time, rapid drying, even at low temperatures and even under high atmospheric humidity. Typical industrial applications for the curing compositions of the invention include, for example, use for the production of shaped articles (casting resins) for tool construction, or for the production of coatings and/or intermediate coatings on many types of substrates, for example, on those of an organic or inorganic nature, such as wood, wood fibers (wood sealing), textiles of natural or synthetic origin, plastics, glass, ceramic and building materials, such as concrete, fiberboards and artificial stones, but in particular on metals, such as optionally pretreated sheet steel, cast iron, aluminum and nonferrous metals, such as brass, bronze and copper. The curing compositions according to the invention can furthermore be employed as constituents of adhesives, putties, laminating resins and synthetic resin cements, and in particular as constituents of paints and coatings for coating industrial objects, domestic appliances and furniture and in the shipbuilding industry, land storage tanks and pipelines and in the building industry, such as, for example, refrigerators, washing machines, electrical appliances, windows and doors.

They can be applied, for example, by brushing, spraying, dipping and the like. A particularly preferred field of use for the composition according to the invention is paints formulations.

The invention will be more readily understood by reference to the following examples and figures, which are included merely for purposes of illustration of certain aspects and embodiments of the present invention and are not intended to limit the invention. The first example relates to the synthesis of a compound according to a preferred embodiment of the invention. The second example relates to the properties of coatings prepared with the compound of example 1. Examples of compound according to the invention are further listed in examples 3 and 4, and example 5 relates to alternate methods for the synthesis of the compounds according to the invention.

### Brief description of the figures

**Figure 1** represents the ¹H NMR spectrum of N,N-(diaminopropyl)4-(2-aminoethyl)phenol.
**Figure 2** represents the ¹³C NMR spectrum of N,N-(diaminopropyl)4-(2-aminoethyl)phenol.

### Examples

### Example 1: Synthesis of N,N-(diaminopropyl)4-(2-aminoethyl)phenol illustrated in scheme 1.

25 g of tyramine (0.1825 mole, tyramine base > 97%, purchased from Calaire Chimie, S.A.) were mixed with 19.35 g of acrylonitrile (0.3651 mole) and 44.3 g of methanol. The reaction mixture was allowed to react 16 h under reflux. Evaporation under reduced pressure yielded 44.3 g of the pure dinitrile derivative (N,N-(dicyanopropyl)4-(2-aminoethyl)phenol, C14H17N3O).

The dinitrile derivative was characterised by NMR:
¹H NMR (300 MHz, ppm): 7.08-7.05 (2H, aromatic); 6.78-6.75 (2H, aromatic); 6-4 (1H, large OH); 2.92-2.88 (4H, m, CH₂-N); 2.78-2.69 (4H, m); 2.49-2.39 (4H, m, CH₂CN).
¹³C NMR (ppm): 154.16; 131.45 (x2); 129.87 (x2) (aromatic C); 118.58 (x2); 115.43 (x2); 55.86; 49.9. (x2); 33.42; 17.18 (x2).
42.7 g of dinitrile derivative was diluted in 105 ml of ethanol. 1g of platinum (IV) oxide (m.p. 450 °C) and 50 ml of concentrated ammonia solution (25% in weight) were added. The mixture was allowed to react in a Parr apparatus under stirring at room temperature at an initial hydrogen pressure of 4 hPa. After 24 h, the pressure dropped to 1 hPa, and was increased again at 3.7 hPa. After 48 h, the pressure dropped to 2.8 hPa and the reaction was stopped. Filtration of the catalyst, subsequent evaporation of the solvent and drying of the product yielded 40.3 g of primary diamine (N,N-(diaminopropyl)4-(2-aminoethyl)phenol, C14h25N30). Its total amine value (according to method ASTM D2073-92) was 456 mg KOH/g.

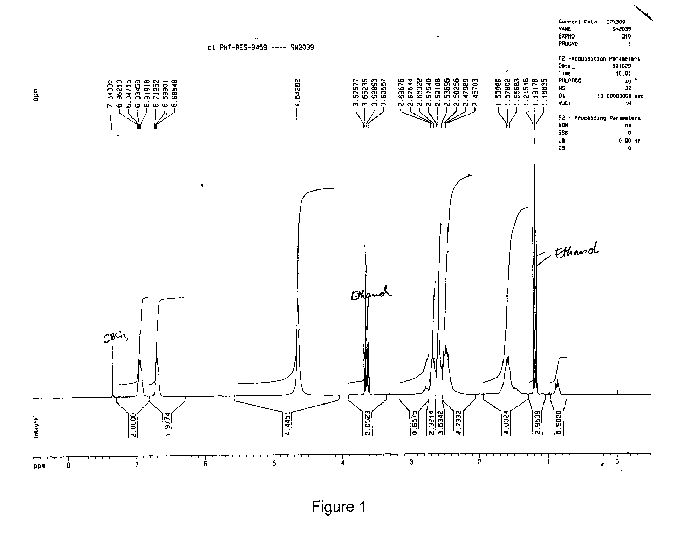

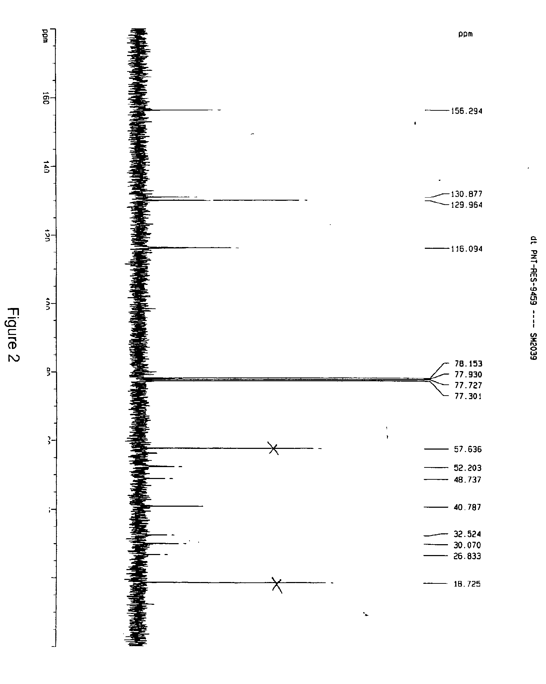

The final product was characterised by NMR as illustrated on Figure 1 and 2:
¹H NMR (300 MHz, ppm) (Figure 1): 6.96-6.92 (2H, aromatic); 6.71-6.69 (2H, aromatic); 4.64 (5H, large, 2 NH₂ + OH); 2.70-2.46 (12H, m); 1.56-1.60 (4H, m).

Peaks at 7.34, 3.67-3.60, 1.21-1.17 and 0.9 ppm are impurities, respectively attributable to chloroform, ethanol and (vacuum) grease.
¹³C NMR (75 MHz, ppm) (Figure 2): 154.29; 130.88; 129.96 (aromatic CH); 116.09 (aromatic CH); 77.93; 52.2; 40.79; 32.52, 30.07.

Peaks at 78-77, 57.6 and 18.7 ppm are attributable to deuterated chloroform and ethanol.

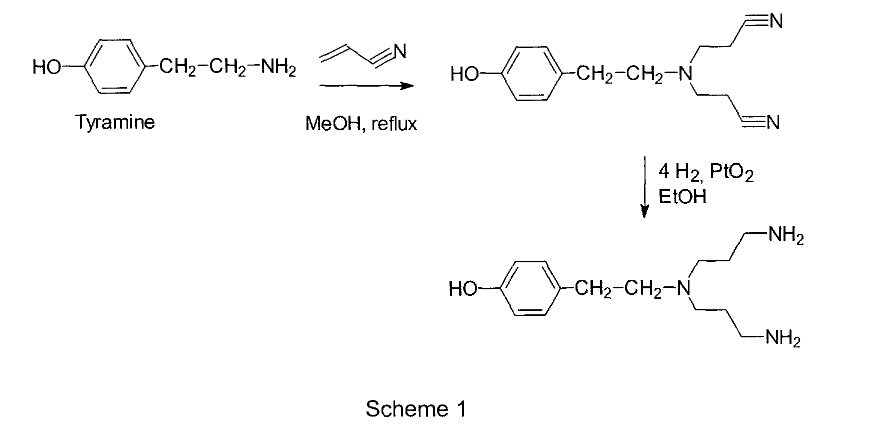

### Example 2: Use of N,N-(diaminopropyl)4-(2-aminoethyl)phenol in an epoxy coating system, as an accelerator.

The hardener base used in the coatings was an adduct between bisphenol A diglycidylether (DGEBA, Epikote 828, Shell) and isophorone diamine (IPD, Hüls Chemie AG), with an amine value of 264 mg KOH/g. The epoxy resin base used in this example was bisphenol A diglycidylether (DBEBA, Epikote 828, Shell).

The quantities of components used in the coatings are shown in Table 1. The components were mixed together. The drawdowns were performed on glass with a bird applicator BA30.

The films were stored at 5 °C and the development of the hardness (König) was monitored during 60 days. The results are summarised in Table 2. The König hardness (ISO1522 and DIN53157) is a pendulum damping test for assessment of the hardness of a coating. A pendulum of particular shape and time of oscillation rests on two balls on the paint film and is set into motion from a certain starting deflection angle (from 6° to 3°). The time in which the pendulum has arrived at a certain final angle is a measurement for the hardness of the paint film. The harder the coated surface, the higher the number of oscillations. The number of oscillations is then converted in seconds.

**Table 1**

| Weights (g) | Reference coating | coating 1 | coating 2 |
|---|---|---|---|
| DGEBA | 100 | 100 | 100 |
| Hardener | 37.89 | 32.93 | 34.86 |
| N,N-(diamino propyl)4-(2-amino ethyl)phenol | - | 2.86 | 3.03 |

**Table 2**

| | Reference coating | coating 1 | coating 2 |
|---|---|---|---|
| After drying at 5 °C: König -hardness after 1-60 days.(in seconds) | | | |
| 1 | 3 | 4 | 3 |
| 2 | 1 | 4 | 6 |
| 3 | 8 | 7 | 13 |
| 4 | 15 | 14 | 18 |
| 7 | 25 | 42 | 31 |
| 10 | 28 | 56 | 34 |
| 15 | 42 | 66 | 57 |
| 30 | 66 | 78 | 57 |
| 60 | 74 | | 167 |

Coatings 1 and 2 exhibited better hardness development and this was clearly visible as early as after 7 days drying at 5°C (compared to the reference coating). The hardness after 30-60 days was clearly improved, showing that the systems reached a better cross-linked network than the reference coating; this means that the reaction between the epoxy resin and the hardener was more complete. After 2 days the through cure of coatings 1 and 2 was almost twice as fast than the coating without use of accelerator. This doubling of Königs hardness became even more obvious after 15 days curing at 5 °C. The cross-linked network resulted in better properties like for instance chemical resistance.

### Example 3: Examples of compounds obtainable by a method according to the invention are listed hereunder.

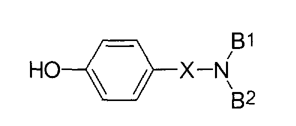

| **Nber** | **X** | **B**^{**1**} | **B**^{**2**} |
|---|---|---|---|
| 1a | -CH₂- | -CH₂-CH₂-NH₂ | -CH₂-CH₂-NH₂ |
| 1b | -CH₂-CH₂- | -CH₂-CH₂-NH₂ | -CH₂-CH₂-NH₂ |
| 1c | -CH₂-CH₂-CH₂- | -CH₂-CH₂-NH₂ | -CH₂-CH₂-NH₂ |
| 1d | -CH₂-CH(OH)- | -CH₂-CH₂-NH₂ | -CH₂-CH₂-NH₂ |
| 1e | -CH(OH)-CH₂- | -CH₂-CH₂-NH₂ | -CH₂-CH₂-NH₂ |
| 1f | -CH₂-CH(CH₃)- | -CH₂-CH₂-NH₂ | -CH₂-CH₂-NH₂ |
| 1g | -CH(CH₃)-CH₂- | -CH₂-CH₂-NH₂ | -CH₂-CH₂-NH₂ |
| 1h | -CH(CH₃)-CH(OH)- | -CH₂-CH₂-NH₂ | -CH₂-CH₂-NH₂ |
| 1l | -CH(OH)-CH(CH₃)- | -CH₂-CH₂-NH₂ | -CH₂-CH₂-NH₂ |
| 1j | -CH₂-O-CH₂- | -CH₂-CH₂-NH₂ | -CH₂-CH₂-NH₂ |
| 1k | -O-CH₂- | -CH₂-CH₂-NH₂ | -CH₂-CH₂-NH₂ |
| 1l | -O-CH₂-CH₂- | -CH₂-CH₂-NH₂ | -CH₂-CH₂-NH₂ |
| 1m | -CH₂-S-CH₂- | -CH₂-CH₂-NH₂ | -CH₂-CH₂-NH₂ |
| 1n | -S-CH₂- | -CH₂-CH₂-NH₂ | -CH₂-CH₂-NH₂ |
| 1o | -CH(CO₂H)- | -CH₂-CH₂-NH₂ | -CH₂-CH₂-NH₂ |
| 1p | -CH(CO₂Me)- | -CH₂-CH₂-NH₂ | -CH₂-CH₂-NH₂ |
| 1q | -CH₂-CH(CO₂H)- | -CH₂-CH₂-NH₂ | -CH₂-CH₂-NH₂ |
| 1r | -CH(OH)-CH(CO₂H)- | -CH₂-CH₂-NH₂ | -CH₂-CH₂-NH₂ |
| 2a | -CH₂- | -CH₂-CH₂-CH₂-NH₂ | -CH₂-CH₂-CH₂-NH₂ |
| 2b | -CH₂-CH₂- | -CH₂-CH₂-CH₂-NH₂ | -CH₂-CH₂-CH₂-NH₂ |
| 2c | -CH₂-CH₂-CH₂- | -CH₂-CH₂-CH₂-NH₂ | -CH₂-CH₂-CH₂-NH₂ |
| 2d | -CH₂-CH(OH)- | -CH₂-CH₂-CH₂-NH₂ | -CH₂-CH₂-CH₂-NH₂ |
| 2e | -CH(OH)-CH₂- | -CH₂-CH₂-CH₂-NH₂ | -CH₂-CH₂-CH₂-NH₂ |
| 2f | -CH₂-CH(CH₃)- | -CH₂-CH₂-CH₂-NH₂ | -CH₂-CH₂-CH₂-NH₂ |
| 2g | -CH(CH₃)-CH₂- | -CH₂-CH₂-CH₂-NH₂ | -CH₂-CH₂-CH₂-NH₂ |
| 2h | -CH(CH₃)-CH(OH)- | -CH₂-CH₂-CH₂-NH₂ | -CH₂-CH₂-CH₂-NH₂ |
| 2i | -CH(OH)-CH(CH₃)- | -CH₂-CH₂-CH₂-NH₂ | -CH₂-CH₂-CH₂-NH₂ |
| 2j | -CH₂-O-CH₂- | -CH₂-CH₂-CH₂-NH₂ | -CH₂-CH₂-CH₂-NH₂ |
| 2k | -O-CH2- | -CH₂-CH₂-CH₂-NH₂ | -CH₂-CH₂-CH₂-NH₂ |
| 2l | -O-CH₂-CH₂- | -CH₂-CH₂-CH₂-NH₂ | -CH₂-CH₂-CH₂-NH₂ |
| 2m | -CH₂-S-CH₂- | -CH₂-CH₂-CH₂-NH₂ | -CH₂-CH₂-CH₂-NH₂ |
| 2n | -S-CH₂- | -CH₂-CH₂-CH₂-NH₂ | -CH₂-CH₂-CH₂-NH₂ |
| 2o | -CH(CO₂H)- | -CH₂-CH₂-CH₂-NH₂ | -CH₂-CH₂-CH₂-NH₂ |
| 2p | -CH(CO₂Me)- | -CH₂-CH₂-CH₂-NH₂ | -CH₂-CH₂-CH₂-NH₂ |
| 2q | -CH₂-CH(CO₂H)- | -CH₂-CH₂-CH₂-NH₂ | -CH₂-CH₂-CH₂-NH₂ |
| 2r | -CH(OH)-CH(CO₂H)- | -CH₂-CH₂-CH₂-NH₂ | -CH₂-CH₂-CH₂-NH₂ |
| 3a | -CH₂- | -CH₂-CH₂-CH₂-NH₂ | -CH₂-CH(CH₃)-CH₂-NH₂ |
| 3b | -CH₂-CH₂- | -CH₂-CH₂-CH₂-NH₂ | -CH₂-CH(CH₃)-CH₂-NH₂ |
| 3c | -CH₂-CH₂-CH₂- | -CH₂-CH₂-CH₂-NH₂ | -CH₂-CH(CH₃)-CH₂-NH₂ |
| 3d | -CH₂-CH(OH)- | -CH₂-CH₂-CH₂-NH₂ | -CH₂-CH(CH₃)-CH₂-NH₂ |
| 3e | -CH(OH)-CH₂- | -CH₂-CH₂-CH₂-NH₂ | -CH₂-CH(CH₃)-CH₂-NH₂ |
| 3f | -CH₂-CH(CH₃)- | -CH₂-CH₂-CH₂-NH₂ | -CH₂-CH(CH₃)-CH₂-NH₂ |
| 3g | -CH(CH₃)-CH₂- | -CH₂-CH₂-CH₂-NH₂ | -CH₂-CH(CH₃)-CH₂-NH₂ |
| 3h | -CH(CH₃)-CH(OH)- | -CH₂-CH₂-CH₂-NH₂ | -CH₂-CH(CH₃)-CH₂-NH₂ |
| 3l | -CH(OH)-CH(CH₃)- | -CH₂-CH₂-CH₂-NH₂ | -CH₂-CH(CH₃)-CH₂-NH₂ |
| 3j | -CH₂-O-CH₂- | -CH₂-CH₂-CH₂-NH₂ | -CH₂-CH(CH₃)-CH₂-NH₂ |
| 3k | -O-CH₂- | -CH₂-CH₂-CH₂-NH₂ | -CH₂-CH(CH₃)-CH₂-NH₂ |
| 3l | -O-CH₂-CH₂- | -CH₂-CH₂-CH₂-NH₂ | -CH₂-CH(CH₃)-CH₂-NH₂ |
| 3m | -CH₂-S-CH₂- | -CH₂-CH₂-CH₂-NH₂ | -CH₂-CH(CH₃)-CH₂-NH₂ |
| 3n | -S-CH₂- | -CH₂-CH₂-CH₂-NH₂ | -CH₂-CH(CH₃)-CH₂-NH₂ |
| 3o | -CH(CO₂H)- | -CH₂-CH₂-CH₂-NH₂ | -CH₂-CH(CH₃)-CH₂-NH₂ |
| 3p | -CH(CO₂Me)- | -CH₂-CH₂-CH₂-NH₂ | -CH₂-CH(CH₃)-CH₂-NH₂ |
| 3q | -CH₂-CH(CO₂H)- | -CH₂-CH₂-CH₂-NH₂ | -CH₂-CH(CH₃)-CH₂-NH₂ |
| 3r | -CH(OH)-CH(CO₂H)- | -CH₂-CH₂-CH₂-NH₂ | -CH₂-CH(CH₃)-CH₂-NH₂ |
| 4a | -CH₂- | -CH₂-CH(CH₃)-CH₂-NH₂ | -CH₂-CH₂-CH₂-NH₂ |
| 4b | -CH₂-CH₂- | -CH₂-CH(CH₃)-CH₂-NH₂ | -CH₂-CH₂-CH₂-NH₂ |
| 4c | -CH₂-CH₂-CH₂- | -CH₂-CH(CH₃)-CH₂-NH₂ | -CH₂-CH₂-CH₂-NH₂ |
| 4d | -CH₂-CH(OH)- | -CH₂-CH(CH₃)-CH₂-NH₂ | -CH₂-CH₂-CH₂-NH₂ |
| 4e | -CH(OH)-CH₂- | -CH₂-CH(CH₃)-CH₂-NH₂ | -CH₂-CH₂-CH₂-NH₂ |
| 4f | -CH₂-CH(CH₃)- | -CH₂-CH(CH₃)-CH₂-NH₂ | -CH₂-CH₂-CH₂-NH₂ |
| 4g | -CH(CH₃)-CH₂- | -CH₂-CH(CH₃)-CH₂-NH₂ | -CH₂-CH₂-CH₂-NH₂ |
| 4h | -CH(CH₃)-CH(OH)- | -CH₂-CH(CH₃)-CH₂-NH₂ | -CH₂-CH₂-CH₂-NH₂ |
| 4i | -CH(OH)-CH(CH₃)- | -CH₂-CH(CH₃)-CH₂-NH₂ | -CH₂-CH₂-CH₂-NH₂ |
| 4j | -CH₂-O-CH₂- | -CH₂-CH(CH₃)-CH₂-NH₂ | -CH₂-CH₂-CH₂-NH₂ |
| 4k | -O-CH₂- | -CH₂-CH(CH₃)-CH₂-NH₂ | -CH₂-CH₂-CH₂-NH₂ |
| 4l | -O-CH₂-CH₂- | -CH₂-CH(CH₃)-CH₂-NH₂ | -CH₂-CH₂-CH₂-NH₂ |
| 4m | -CH₂-S-CH₂- | -CH₂-CH(CH₃)-CH₂-NH₂ | -CH₂-CH₂-CH₂-NH₂ |
| 4n | -S-CH₂- | -CH₂-CH(CH₃)-CH₂-NH₂ | -CH₂-CH₂-CH₂-NH₂ |
| 4o | -CH(CO₂H)- | -CH₂-CH(CH₃)-CH₂-NH₂ | -CH₂-CH₂-CH₂-NH₂ |
| 4p | -CH(CO₂Me)- | -CH₂-CH(CH₃)-CH₂-NH₂ | -CH₂-CH₂-CH₂-NH₂ |
| 4q | -CH₂-CH(CO₂H)- | -CH₂-CH(CH₃)-CH₂-NH₂ | -CH₂-CH₂-CH₂-NH₂ |
| 4r | -CH(OH)-CH(CO₂H)- | -CH₂-CH(CH₃)-CH₂-NH₂ | -CH₂-CH₂-CH₂-NH₂ |
| 5a | -CH₂- | -CH₂-CH(CH₃)-CH₂-NH₂ | -CH₂-CH(CH₃)-CH₂-NH₂ |
| 5b | -CH₂-CH₂- | -CH₂-CH(CH₃)-CH₂-NH₂ | -CH₂-CH(CH₃)-CH₂-NH₂ |
| 5c | -CH₂-CH₂-CH₂- | -CH₂-CH(CH₃)-CH₂-NH₂ | -CH₂-CH(CH₃)-CH₂-NH₂ |
| 5d | -CH₂-CH(OH)- | -CH₂-CH(CH₃)-CH₂-NH₂ | -CH₂-CH(CH₃)-CH₂-NH₂ |
| 5e | -CH(OH)-CH₂- | -CH₂-CH(CH₃)-CH₂-NH₂ | -CH₂-CH(CH₃)-CH₂-NH₂ |
| 5f | -CH₂-CH(CH₃)- | -CH₂-CH(CH₃)-CH₂-NH₂ | -CH₂-CH(CH₃)-CH₂-NH₂ |
| 5g | -CH(CH₃)-CH₂- | -CH₂-CH(CH₃)-CH₂-NH₂ | -CH₂-CH(CH₃)-CH₂-NH₂ |
| 5h | -CH(CH₃)-CH(OH)- | -CH₂-CH(CH₃)-CH₂-NH₂ | -CH₂-CH(CH₃)-CH₂-NH₂ |
| 5i | -CH(OH)-CH(CH₃)- | -CH₂-CH(CH₃)-CH₂-NH₂ | -CH₂-CH(CH₃)-CH₂-NH₂ |
| 5j | -CH₂-O-CH₂- | -CH₂-CH(CH₃)-CH₂-NH₂ | -CH₂-CH(CH₃)-CH₂-NH₂ |
| 5k | -O-CH₂- | -CH₂-CH(CH₃)-CH₂-NH₂ | -CH₂-CH(CH₃)-CH₂-NH₂ |
| 5l | -O-CH₂-CH₂- | -CH₂-CH(CH₃)-CH₂-NH₂ | -CH₂-CH(CH₃)-CH₂-NH₂ |
| 5m | -CH₂-S-CH₂- | -CH₂-CH(CH₃)-CH₂-NH₂ | -CH₂-CH(CH₃)-CH₂-NH₂ |
| 5n | -S-CH₂- | -CH₂-CH(CH₃)-CH₂-NH₂ | -CH₂-CH(CH₃)-CH₂-NH₂ |
| 5o | -CH(CO₂H)- | -CH₂-CH(CH₃)-CH₂-NH₂ | -CH₂-CH(CH₃)-CH₂-NH₂ |
| 5p | -CH(CO₂Me)- | -CH₂-CH(CH₃)-CH₂-NH₂ | -CH₂-CH(CH₃)-CH₂-NH₂ |
| 5q | -CH₂-CH(CO₂H)- | -CH₂-CH(CH₃)-CH₂-NH₂ | -CH₂-CH(CH₃)-CH₂-NH₂ |
| 5r | -CH(OH)-CH(CO₂H)- | -CH₂-CH(CH₃)-CH₂-NH₂ | -CH₂-CH(CH₃)-CH₂-NH₂ |
| 6a | -CH₂- | H | -CH₂-CH₂-NH₂ |
| 6b | -CH₂-CH₂- | H | -CH₂-CH₂-NH₂ |
| 6c | -CH₂-CH₂-CH₂- | H | -CH₂-CH₂-NH₂ |
| 6d | -CH₂-CH(OH)- | H | -CH₂-CH₂-NH₂ |
| 6e | -CH(OH)-CH₂- | H | -CH₂-CH₂-NH₂ |
| 6f | -CH₂-CH(CH₃)- | H | -CH₂-CH₂-NH₂ |
| 6g | -CH(CH₃)-CH₂- | H | -CH₂-CH₂-NH₂ |
| 6h | -CH(CH₃)-CH(OH)- | H | -CH₂-CH₂-NH₂ |
| 6l | -CH(OH)-CH(CH₃)- | H | -CH₂-CH₂-NH₂ |
| 6j | -CH₂-O-CH₂- | H | -CH₂-CH₂-NH₂ |
| 6k | -O-CH₂- | H | -CH₂-CH₂-NH₂ |
| 6l | -O-CH₂-CH₂- | H | -CH₂-CH₂-NH₂ |
| 6m | -CH₂-S-CH₂- | H | -CH₂-CH₂-NH₂ |
| 6n | -S-CH₂- | H | -CH₂-CH₂-NH₂ |
| 6o | -CH(CO₂H)- | H | -CH₂-CH₂-NH₂ |
| 6p | -CH(CO₂Me)- | H | -CH₂-CH₂-NH₂ |
| 6q | -CH₂-CH(CO₂H)- | H | -CH₂-CH₂-NH₂ |
| 6r | -CH(OH)-CH(CO₂H)- | H | -CH₂-CH₂-NH₂ |
| 7a | -CH₂- | H | -CH₂-CH₂-CH₂-NH₂ |
| 7b | -CH₂-CH₂- | H | -CH₂-CH₂-CH₂-NH₂ |
| 7c | -CH₂-CH₂-CH₂- | H | -CH₂-CH₂-CH₂-NH₂ |
| 7d | -CH₂-CH(OH)- | H | -CH₂-CH₂-CH₂-NH₂ |
| 7e | -CH(OH)-CH₂- | H | -CH₂-CH₂-CH₂-NH₂ |
| 7f | -CH₂-CH(CH₃)- | H | -CH₂-CH₂-CH₂-NH₂ |
| 7g | -CH(CH₃)-CH₂- | H | -CH₂-CH₂-CH₂-NH₂ |
| 7h | -CH(CH₃)-CH(OH)- | H | -CH₂-CH₂-CH₂-NH₂ |
| 7l | -CH(OH)-CH(CH₃)- | H | -CH₂-CH₂-CH₂-NH₂ |
| 7j | -CH₂-O-CH₂- | H | -CH₂-CH₂-CH₂-NH₂ |
| 7k | -O-CH₂- | H | -CH₂-CH₂-CH₂-NH₂ |
| 7l | -O-CH₂-CH₂- | H | -CH₂-CH₂-CH₂-NH₂ |
| 7m | -CH₂-S-CH₂- | H | -CH₂-CH₂-CH₂-NH₂ |
| 7n | -S-CH₂- | H | -CH₂-CH₂-CH₂-NH₂ |
| 7o | -CH(CO₂H)- | H | -CH₂-CH₂-CH₂-NH₂ |
| 7p | -CH(CO₂Me)- | H | -CH₂-CH₂-CH₂-NH₂ |
| 7q | -CH₂-CH(CO₂H)- | H | -CH₂-CH₂-CH₂-NH₂ |
| 7r | -CH(OH)-CH(CO₂H)- | H | -CH₂-CH₂-CH₂-NH₂ |
| 8a | -CH₂- | H | -CH₂-CH(CH₃)-CH₂-NH₂ |
| 8b | -CH₂-CH₂- | H | -CH₂-CH(CH₃)-CH₂-NH₂ |
| 8c | -CH₂-CH₂-CH₂- | H | -CH₂-CH(CH₃)-CH₂-NH₂ |
| 8d | -CH₂-CH(OH)- | H | -CH₂-CH(CH₃)-CH₂-NH₂ |
| 8e | -CH(OH)-CH₂- | H | -CH₂-CH(CH₃)-CH₂-NH₂ |
| 8f | -CH₂-CH(CH₃)- | H | -CH₂-CH(CH₃)-CH₂-NH₂ |
| 8g | -CH(CH₃)-CH₂- | H | -CH₂-CH(CH₃)-CH₂-NH₂ |
| 8h | -CH(CH₃)-CH(OH)- | H | -CH₂-CH(CH₃)-CH₂-NH₂ |
| 8i | -CH(OH)-CH(CH₃)- | H | -CH₂-CH(CH₃)-CH₂-NH₂ |
| 8j | -CH₂-O-CH₂- | H | -CH₂-CH(CH₃)-CH₂-NH₂ |
| 8k | -O-CH₂- | H | -CH₂-CH(CH₃)-CH₂-NH₂ |
| 8l | -O-CH₂-CH₂- | H | -CH₂-CH(CH₃)-CH₂-NH₂ |
| 8m | -CH₂-S-CH₂- | H | -CH₂-CH(CH₃)-CH₂-NH₂ |
| 8n | -S-CH₂- | H | -CH₂-CH(CH₃)-CH₂-NH₂ |
| 8o | -CH(CO₂H)- | H | -CH₂-CH(CH₃)-CH₂-NH₂ |
| 8p | -CH(CO₂Me)- | H | -CH₂-CH(CH₃)-CH₂-NH₂ |
| 8q | -CH₂-CH(CO₂H)- | H | -CH₂-CH(CH₃)-CH₂-NH₂ |
| 8r | -CH(OH)-CH(CO₂H)- | H | -CH₂-CH(CH₃)-CH₂-NH₂ |

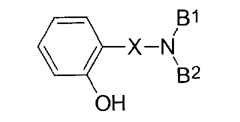

| **Nber** | **X** | **B**^{**1**} | **B**^{**2**} |
|---|---|---|---|
| 9a | -CH₂- | -CH₂-CH₂-NH₂ | -CH₂-CH₂-NH₂ |
| 9b | -CH₂-CH₂- | -CH₂-CH₂-NH₂ | -CH₂-CH₂-NH₂ |
| 9c | -CH₂-CH₂-CH₂- | -CH₂-CH₂-NH₂ | -CH₂-CH₂-NH₂ |
| 9d | -CH₂-CH(OH)- | -CH₂-CH₂-NH₂ | -CH₂-CH₂-NH₂ |
| 9e | -CH(OH)-CH₂- | -CH₂-CH₂-NH₂ | -CH₂-CH₂-NH₂ |
| 9f | -CH₂-CH(CH₃)- | -CH₂-CH₂-NH₂ | -CH₂-CH₂-NH₂ |
| 9g | -CH(CH₃)-CH₂- | -CH₂-CH₂-NH₂ | -CH₂-CH₂-NH₂ |
| 9h | -CH(CH₃)-CH(OH)- | -CH₂-CH₂-NH₂ | -CH₂-CH₂-NH₂ |
| 9i | -CH(OH)-CH(CH₃)- | -CH₂-CH₂-NH₂ | -CH₂-CH₂-NH₂ |
| 9j | -CH₂-O-CH₂- | -CH₂-CH₂-NH₂ | -CH₂-CH₂-NH₂ |
| 9k | -O-CH₂- | -CH₂-CH₂-NH₂ | -CH₂-CH₂-NH₂ |
| 9l | -O-CH₂-CH₂- | -CH₂-CH₂-NH₂ | -CH₂-CH₂-NH₂ |
| 9m | -CH₂-S-CH₂- | -CH₂-CH₂-NH₂ | -CH₂-CH₂-NH₂ |
| 9n | -S-CH₂- | -CH₂-CH₂-NH₂ | -CH₂-CH₂-NH₂ |
| 9o | -CH(CO₂H)- | -CH₂-CH₂-NH₂ | -CH₂-CH₂-NH₂ |
| 9p | -CH(CO₂Me)- | -CH₂-CH₂-NH₂ | -CH₂-CH₂-NH₂ |
| 9q | -CH₂-CH(CO₂H)- | -CH₂-CH₂-NH₂ | -CH₂-CH₂-NH₂ |
| 9r | -CH(OH)-CH(CO₂H)- | -CH₂-CH₂-NH₂ | -CH₂-CH₂-NH₂ |
| 10a | -CH₂- | -CH₂-CH₂-CH₂-NH₂ | -CH₂-CH₂-CH₂-NH₂ |
| 10b | -CH₂-CH₂- | -CH₂-CH₂-CH₂-NH₂ | -CH₂-CH₂-CH₂-NH₂ |
| 10c | -CH₂-CH₂-CH₂- | -CH₂-CH₂-CH₂-NH₂ | -CH₂-CH₂-CH₂-NH₂ |
| 10d | -CH₂-CH(OH)- | -CH₂-CH₂-CH₂-NH₂ | -CH₂-CH₂-CH₂-NH₂ |
| 10e | -CH(OH)-CH₂- | -CH₂-CH₂-CH₂-NH₂ | -CH₂-CH₂-CH₂-NH₂ |
| 10f | -CH₂-CH(CH₃)- | -CH₂-CH₂-CH₂-NH₂ | -CH₂-CH₂-CH₂-NH₂ |
| 10g | -CH(CH₃)-CH₂- | -CH₂-CH₂-CH₂-NH₂ | -CH₂-CH₂-CH₂-NH₂ |
| 10h | -CH(CH₃)-CH(OH)- | -CH₂-CH₂-CH₂-NH₂ | -CH₂-CH₂-CH₂-NH₂ |
| 10i | -CH(OH)-CH(CH₃)- | -CH₂-CH₂-CH₂-NH₂ | -CH₂-CH₂-CH₂-NH₂ |
| 10j | -CH₂-O-CH₂- | -CH₂-CH₂-CH₂-NH₂ | -CH₂-CH₂-CH₂-NH₂ |
| 10k | -O-CH₂- | -CH₂-CH₂-CH₂-NH₂ | -CH₂-CH₂-CH₂-NH₂ |
| 10l | -O-CH₂-CH₂- | -CH₂-CH₂-CH₂-NH₂ | -CH₂-CH₂-CH₂-NH₂ |
| 10m | -CH₂-S-CH₂- | -CH₂-CH₂-CH₂-NH₂ | -CH₂-CH₂-CH₂-NH₂ |
| 10n | -S-CH₂- | -CH₂-CH₂-CH₂-NH₂ | -CH₂-CH₂-CH₂-NH₂ |
| 100 | -CH(CO₂H)- | -CH₂-CH₂-CH₂-NH₂ | -CH₂-CH₂-CH₂-NH₂ |
| 10p | -CH(CO₂Me)- | -CH₂-CH₂-CH₂-NH₂ | -CH₂-CH₂-CH₂-NH₂ |
| 10q | -CH₂-CH(CO₂H)- | -CH₂-CH₂-CH₂-NH₂ | -CH₂-CH₂-CH₂-NH₂ |
| 10r | -CH(OH)-CH(CO₂H)- | -CH₂-CH₂-CH₂-NH₂ | -CH₂-CH₂-CH₂-NH₂ |
| 11a | -CH₂- | -CH₂-CH₂-CH₂-NH₂ | -CH₂-CH(CH₃)-CH₂-NH₂ |
| 11b | -CH₂-CH₂- | -CH₂-CH₂-CH₂-NH₂ | -CH₂-CH(CH₃)-CH₂-NH₂ |
| 11c | -CH₂-CH₂-CH₂- | -CH₂-CH₂-CH₂-NH₂ | -CH₂-CH(CH₃)-CH₂-NH₂ |
| 11d | -CH₂-CH(OH)- | -CH₂-CH₂-CH₂-NH₂ | -CH₂-CH(CH₃)-CH₂-NH₂ |
| 11e | -CH(OH)-CH₂- | -CH₂-CH₂-CH₂-NH₂ | -CH₂-CH(CH₃)-CH₂-NH₂ |
| 11f | -CH₂-CH(CH₃)- | -CH₂-CH₂-CH₂-NH₂ | -CH₂-CH(CH₃)-CH₂-NH₂ |
| 11g | -CH(CH₃)-CH₂- | -CH₂-CH₂-CH₂-NH₂ | -CH₂-CH(CH₃)-CH₂-NH₂ |
| 11h | -CH(CH₃)-CH(OH)- | -CH₂-CH₂-CH₂-NH₂ | -CH₂-CH(CH₃)-CH₂-NH₂ |
| 11i | -CH(OH)-CH(CH₃)- | -CH₂-CH₂-CH₂-NH₂ | -CH₂-CH(CH₃)-CH₂-NH₂ |
| 11j | -CH₂-O-CH₂- | -CH₂-CH₂-CH₂-NH₂ | -CH₂-CH(CH₃)-CH₂-NH₂ |
| 11k | -O-CH₂- | -CH₂-CH₂-CH₂-NH₂ | -CH₂-CH(CH₃)-CH₂-NH₂ |
| 11l | -O-CH₂-CH₂- | -CH₂-CH₂-CH₂-NH₂ | -CH₂-CH(CH₃)-CH₂-NH₂ |
| 11m | -CH₂-S-CH₂- | -CH₂-CH₂-CH₂-NH₂ | -CH₂-CH(CH₃)-CH₂-NH₂ |
| 11n | -S-CH₂- | -CH₂-CH₂-CH₂-NH₂ | -CH₂-CH(CH₃)-CH₂-NH₂ |
| 11o | -CH(CO₂H)- | -CH₂-CH₂-CH₂-NH₂ | -CH₂-CH(CH₃)-CH₂-NH₂ |
| 11p | -CH(CO₂Me)- | -CH₂-CH₂-CH₂-NH₂ | -CH₂-CH(CH₃)-CH₂-NH₂ |
| 11q | -CH₂-CH(CO₂H)- | -CH₂-CH₂-CH₂-NH₂ | -CH₂-CH(CH₃)-CH₂-NH₂ |
| 11r | -CH(OH)-CH(CO₂H)- | -CH₂-CH₂-CH₂-NH₂ | -CH₂-CH(CH₃)-CH₂-NH₂ |
| 12a | -CH₂- | -CH₂-CH(CH₃)-CH₂-NH₂ | -CH₂-CH₂-CH₂-NH₂ |
| 12b | -CH₂-CH₂- | -CH₂-CH(CH₃)-CH₂-NH₂ | -CH₂-CH₂-CH₂-NH₂ |
| 12c | -CH₂-CH₂-CH₂- | -CH₂-CH(CH₃)-CH₂-NH₂ | -CH₂-CH₂-CH₂-NH₂ |
| 12d | -CH₂-CH(OH)- | -CH₂-CH(CH₃)-CH₂-NH₂ | -CH₂-CH₂-CH₂-NH₂ |
| 12e | -CH(OH)-CH₂- | -CH₂-CH(CH₃)-CH₂-NH₂ | -CH₂-CH₂-CH₂-NH₂ |
| 12f | -CH₂-CH(CH₃)- | -CH₂-CH(CH₃)-CH₂-NH₂ | -CH₂-CH₂-CH₂-NH₂ |
| 12g | -CH(CH₃)-CH₂- | -CH₂-CH(CH₃)-CH₂-NH₂ | -CH₂-CH₂-CH₂-NH₂ |
| 12h | -CH(CH₃)-CH(OH)- | -CH₂-CH(CH₃)-CH₂-NH₂ | -CH₂-CH₂-CH₂-NH₂ |
| 12i | -CH(OH)-CH(CH₃)- | -CH₂-CH(CH₃)-CH₂-NH₂ | -CH₂-CH₂-CH₂-NH₂ |
| 12j | -CH₂-O-CH₂- | -CH₂-CH(CH₃)-CH₂-NH₂ | -CH₂-CH₂-CH₂-NH₂ |
| 12k | -O-CH₂- | -CH₂-CH(CH₃)-CH₂-NH₂ | -CH₂-CH₂-CH₂-NH₂ |
| 12l | -O-CH₂-CH₂- | -CH₂-CH(CH₃)-CH₂-NH₂ | -CH₂-CH₂-CH₂-NH₂ |
| 12m | -CH₂-S-CH₂- | -CH₂-CH(CH₃)-CH₂-NH₂ | -CH₂-CH₂-CH₂-NH₂ |
| 12n | -S-CH₂- | -CH₂-CH(CH₃)-CH₂-NH₂ | -CH₂-CH₂-CH₂-NH₂ |
| 12o | -CH(CO₂H)- | -CH₂-CH(CH₃)-CH₂-NH₂ | -CH₂-CH₂-CH₂-NH₂ |
| 12p | -CH(CO₂Me)- | -CH₂-CH(CH₃)-CH₂-NH₂ | -CH₂-CH₂-CH₂-NH₂ |
| 12q | -CH₂-CH(CO₂H)- | -CH₂-CH(CH₃)-CH₂-NH₂ | -CH₂-CH₂-CH₂-NH₂ |
| 12r | -CH(OH)-CH(CO₂H)- | -CH₂-CH(CH₃)-CH₂-NH₂ | -CH₂-CH₂-CH₂-NH₂ |
| 13a | -CH₂- | -CH₂-CH(CH₃)-CH₂-NH₂ | -CH₂-CH(CH₃)-CH₂-NH₂ |
| 13b | -CH₂-CH₂- | -CH₂-CH(CH₃)-CH₂-NH₂ | -CH₂-CH(CH₃)-CH₂-NH₂ |
| 13c | -CH₂-CH₂-CH₂- | -CH₂-CH(CH₃)-CH₂-NH₂ | -CH₂-CH(CH₃)-CH₂-NH₂ |
| 13d | -CH₂-CH(OH)- | -CH₂-CH(CH₃)-CH₂-NH₂ | -CH₂-CH(CH₃)-CH₂-NH₂ |
| 13e | -CH(OH)-CH₂- | -CH₂-CH(CH₃)-CH₂-NH₂ | -CH₂-CH(CH₃)-CH₂-NH₂ |
| 13f | -CH₂-CH(CH₃)- | -CH₂-CH(CH₃)-CH₂-NH₂ | -CH₂-CH(CH₃)-CH₂-NH₂ |
| 13g | -CH(CH₃)-CH₂- | -CH₂-CH(CH₃)-CH₂-NH₂ | -CH₂-CH(CH₃)-CH₂-NH₂ |
| 13h | -CH(CH₃)-CH(OH)- | -CH₂-CH(CH₃)-CH₂-NH₂ | -CH₂-CH(CH₃)-CH₂-NH₂ |
| 13i | -CH(OH)-CH(CH₃)- | -CH₂-CH(CH₃)-CH₂-NH₂ | -CH₂-CH(CH₃)-CH₂-NH₂ |
| 13j | -CH₂-O-CH₂- | -CH₂-CH(CH₃)-CH₂-NH₂ | -CH₂-CH(CH₃)-CH₂-NH₂ |
| 13k | -O-CH₂- | -CH₂-CH(CH₃)-CH₂-NH₂ | -CH₂-CH(CH₃)-CH₂-NH₂ |
| 13l | -O-CH₂-CH₂- | -CH₂-CH(CH₃)-CH₂-NH₂ | -CH₂-CH(CH₃)-CH₂-NH₂ |
| 13m | -CH₂-S-CH₂- | -CH₂-CH(CH₃)-CH₂-NH₂ | -CH₂-CH(CH₃)-CH₂-NH₂ |
| 13n | -S-CH₂- | -CH₂-CH(CH₃)-CH₂-NH₂ | -CH₂-CH(CH₃)-CH₂-NH₂ |
| 13o | -CH(CO₂H)- | -CH₂-CH(CH₃)-CH₂-NH₂ | -CH₂-CH(CH₃)-CH₂-NH₂ |
| 13p | -CH(CO₂Me)- | -CH₂-CH(CH₃)-CH₂-NH₂ | -CH₂-CH(CH₃)-CH₂-NH₂ |
| 13q | -CH₂-CH(CO₂H)- | -CH₂-CH(CH₃)-CH₂-NH₂ | -CH₂-CH(CH₃)-CH₂-NH₂ |
| 13r | -CH(OH)-CH(CO₂H)- | -CH₂-CH(CH₃)-CH₂-NH₂ | -CH₂-CH(CH₃)-CH₂-NH₂ |
| 14a | -CH₂- | H | -CH₂-CH₂-NH₂ |
| 14b | -CH₂-CH₂- | H | -CH₂-CH₂-NH₂ |
| 14c | -CH₂-CH₂-CH₂- | H | -CH₂-CH₂-NH₂ |
| 14d | -CH₂-CH(OH)- | H | -CH₂-CH₂-NH₂ |
| 14e | -CH(OH)-CH₂- | H | -CH₂-CH₂-NH₂ |
| 14f | -CH₂-CH(CH₃)- | H | -CH₂-CH₂-NH₂ |
| 14g | -CH(CH₃)-CH₂- | H | -CH₂-CH₂-NH₂ |
| 14h | -CH(CH₃)-CH(OH)- | H | -CH₂-CH₂-NH₂ |
| 14i | -CH(OH)-CH(CH₃)- | H | -CH₂-CH₂-NH₂ |
| 14j | -CH₂-O-CH₂- | H | -CH₂-CH₂-NH₂ |
| 14k | -O-CH₂- | H | -CH₂-CH₂-NH₂ |
| 14l | -O-CH₂-CH₂- | H | -CH₂-CH₂-NH₂ |
| 14m | -CH₂-S-CH₂- | H | -CH₂-CH₂-NH₂ |
| 14n | -S-CH₂- | H | -CH₂-CH₂-NH₂ |
| 14o | -CH(CO₂H)- | H | -CH₂-CH₂-NH₂ |
| 14p | -CH(CO₂Me)- | H | -CH₂-CH₂-NH₂ |
| 14q | -CH₂-CH(CO₂H)- | H | -CH₂-CH₂-NH₂ |
| 14r | -CH(OH)-CH(CO₂H)- | H | -CH₂-CH₂-NH₂ |
| 15a | -CH₂- | H | -CH₂-CH₂-CH₂-NH₂ |
| 15b | -CH₂-CH₂- | H | -CH₂-CH₂-CH₂-NH₂ |
| 15c | -CH₂-CH₂-CH₂- | H | -CH₂-CH₂-CH₂-NH₂ |
| 15d | -CH₂-CH(OH)- | H | -CH₂-CH₂-CH₂-NH₂ |
| 15e | -CH(OH)-CH₂- | H | -CH₂-CH₂-CH₂-NH₂ |
| 15f | -CH₂-CH(CH₃)- | H | -CH₂-CH₂-CH₂-NH₂ |
| 15g | -CH(CH₃)-CH₂- | H | -CH₂-CH₂-CH₂-NH₂ |
| 15h | -CH(CH₃)-CH(OH)- | H | -CH₂-CH₂-CH₂-NH₂ |
| 15i | -CH(OH)-CH(CH₃)- | H | -CH₂-CH₂-CH₂-NH₂ |
| 15j | -CH₂-O-CH₂- | H | -CH₂-CH₂-CH₂-NH₂ |
| 15k | -O-CH₂- | H | -CH₂-CH₂-CH₂-NH₂ |
| 15l | -O-CH₂-CH₂- | H | -CH₂-CH₂-CH₂-NH₂ |
| 15m | -CH₂-S-CH₂- | H | -CH₂-CH₂-CH₂-NH₂ |
| 15n | -S-CH₂- | H | -CH₂-CH₂-CH₂-NH₂ |
| 15o | -CH(CO₂H)- | H | -CH₂-CH₂-CH₂-NH₂ |
| 15p | -CH(CO₂Me)- | H | -CH₂-CH₂-CH₂-NH₂ |
| 15q | -CH₂-CH(CO₂H)- | H | -CH₂-CH₂-CH₂-NH₂ |
| 15r | -CH(OH)-CH(CO₂H)- | H | -CH₂-CH₂-CH₂-NH₂ |
| 16a | -CH₂- | H | -CH₂-CH(CH₃)-CH₂-NH₂ |
| 16b | -CH₂-CH₂- | H | -CH₂-CH(CH₃)-CH₂-NH₂ |
| 16c | -CH₂-CH₂-CH₂- | H | -CH₂-CH(CH₃)-CH₂-NH₂ |
| 16d | -CH₂-CH(OH)- | H | -CH₂-CH(CH₃)-CH₂-NH₂ |
| 16e | -CH(OH)-CH₂- | H | -CH₂-CH(CH₃)-CH₂-NH₂ |
| 16f | -CH₂-CH(CH₃)- | H | -CH₂-CH(CH₃)-CH₂-NH₂ |
| 16g | -CH(CH₃)-CH₂- | H | -CH₂-CH(CH₃)-CH₂-NH₂ |
| 16h | -CH(CH₃)-CH(OH)- | H | -CH₂-CH(CH₃)-CH₂-NH₂ |
| 16i | -CH(OH)-CH(CH₃)- | H | -CH₂-CH(CH₃)-CH₂-NH₂ |
| 16j | -CH₂-O-CH₂- | H | -CH₂-CH(CH₃)-CH₂-NH₂ |
| 16k | -O-CH₂- | H | -CH₂-CH(CH₃)-CH₂-NH₂ |
| 16l | -O-CH₂-CH₂- | H | -CH₂-CH(CH₃)-CH₂-NH₂ |
| 16m | -CH₂-S-CH₂- | H | -CH₂-CH(CH₃)-CH₂-NH₂ |
| 16n | -S-CH₂- | H | -CH₂-CH(CH₃)-CH₂-NH₂ |
| 16o | -CH(CO₂H)- | H | -CH₂-CH(CH₃)-CH₂-NH₂ |
| 16p | -CH(CO₂Me)- | H | -CH₂-CH(CH₃)-CH₂-NH₂ |
| 16q | -CH₂-CH(CO₂H)- | H | -CH₂-CH(CH₃)-CH₂-NH₂ |
| 16r | -CH(OH)-CH(CO₂H)- | H | -CH₂-CH(CH₃)-CH₂-NH₂ |

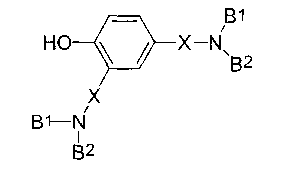

| **Nber** | **X** | **B**^{**1**} | **B**^{**2**} |
|---|---|---|---|
| 17a | -CH₂- | -CH₂-CH₂-NH₂ | -CH₂-CH₂-NH₂ |
| 17b | -CH₂-CH₂- | -CH₂-CH₂-NH_{z} | -CH₂-CH₂-NH₂ |
| 17c | -CH₂-CH₂-CH₂- | -CH₂-CH₂-NH₂ | -CH₂-CH₂-NH₂ |
| 17d | -CH₂-CH(OH)- | -CH₂-CH₂-NH₂ | -CH₂-CH₂-NH₂ |
| 17e | -CH(OH)-CH₂- | -CH₂-CH₂-NH₂ | -CH₂-CH₂-NH₂ |
| 17f | -CH₂-CH(CH₃)- | -CH₂-CH₂-NH₂ | -CH₂-CH₂-NH₂ |
| 17g | -CH(CH₃)-CH₂- | -CH₂-CH₂-NH₂ | -CH₂-CH₂-NH₂ |
| 17h | -CH(CH₃)-CH(OH)- | -CH₂-CH₂-NH₂ | -CH₂-CH₂-NH₂ |
| 17i | -CH(OH)-CH(CH₃)- | -CH₂-CH₂-NH₂ | -CH₂-CH₂-NH₂ |
| 17j | -CH₂-O-CH₂- | -CH₂-CH₂-NH₂ | -CH₂-CH₂-NH₂ |
| 17k | -O-CH₂- | -CH₂-CH₂-NH₂ | -CH₂-CH₂-NH₂ |
| 17l | -O-CH₂-CH₂- | -CH₂-CH₂-NH₂ | -CH₂-CH₂-NH₂ |
| 17m | -CH₂-S-CH₂- | -CH₂-CH₂-NH₂ | -CH₂-CH₂-NH₂ |
| 17n | -S-CH₂- | -CH₂-CH₂-NH₂ | -CH₂-CH₂-NH₂ |
| 17o | -CH(CO₂H)- | -CH₂-CH₂-NH₂ | -CH₂-CH₂-NH₂ |
| 17p | -CH(CO₂Me)- | -CH₂-CH₂-NH₂ | -CH₂-CH₂-NH₂ |
| 17q | -CH₂-CH(CO₂H)- | -CH₂-CH₂-NH₂ | -CH₂-CH₂-NH₂ |
| 17r | -CH(OH)-CH(CO₂H)- | -CH₂-CH₂-NH₂ | -CH₂-CH₂-NH₂ |
| 18a | -CH₂- | -CH₂-CH₂-CH₂-NH₂ | -CH₂-CH₂-CH₂-NH₂ |
| 18b | -CH₂-CH₂- | -CH₂-CH₂-CH₂-NH₂ | -CH₂-CH₂-CH₂-NH₂ |
| 18c | -CH₂-CH₂-CH₂- | -CH₂-CH₂-CH₂-NH₂ | -CH₂-CH₂-CH₂-NH₂ |
| 18d | -CH₂-CH(OH)- | -CH₂-CH₂-CH₂-NH₂ | -CH₂-CH₂-CH₂-NH₂ |
| 18e | -CH(OH)-CH₂- | -CH₂-CH₂-CH₂-NH₂ | -CH₂-CH₂-CH₂-NH₂ |
| 18f | -CH₂-CH(CH₃)- | -CH₂-CH₂-CH₂-NH₂ | -CH₂-CH₂-CH₂-NH₂ |
| 18g | -CH(CH₃)-CH₂- | -CH₂-CH₂-CH₂-NH₂ | -CH₂-CH₂-CH₂-NH₂ |
| 18h | -CH(CH₃)-CH(OH)- | -CH₂-CH₂-CH₂-NH₂ | -CH₂-CH₂-CH₂-NH₂ |
| 18i | -CH(OH)-CH(CH₃)- | -CH₂-CH₂-CH₂-NH₂ | -CH₂-CH₂-CH₂-NH₂ |
| 18j | -CH₂-O-CH₂- | -CH₂-CH₂-CH₂-NH₂ | -CH₂-CH₂-CH₂-NH₂ |
| 18k | -O-CH2- | -CH₂-CH₂-CH₂-NH₂ | -CH₂-CH₂-CH₂-NH₂ |
| 18l | -O-CH₂-CH₂- | -CH₂-CH₂-CH₂-NH₂ | -CH₂-CH₂-CH₂-NH₂ |
| 18m | -CH₂-S-CH₂- | -CH₂-CH₂-CH₂-NH₂ | -CH₂-CH₂-CH₂-NH₂ |
| 18n | -S-CH₂- | -CH₂-CH₂-CH₂-NH₂ | -CH₂-CH₂-CH₂-NH₂ |
| 18o | -CH(CO₂H)- | -CH₂-CH₂-CH₂-NH₂ | -CH₂-CH₂-CH₂-NH₂ |
| 18p | -CH(CO₂Me)- | -CH₂-CH₂-CH₂-NH₂ | -CH₂-CH₂-CH₂-NH₂ |
| 18q | -CH₂-CH(CO₂H)- | -CH₂-CH₂-CH₂-NH₂ | -CH₂-CH₂-CH₂-NH₂ |
| 18r | -CH(OH)-CH(CO₂H)- | -CH₂-CH₂-CH₂-NH₂ | -CH₂-CH₂-CH₂-NH₂ |
| 19a | -CH₂- | -CH₂-CH₂-CH₂-NH₂ | -CH₂-CH(CH₃)-CH₂-NH₂ |
| 19b | -CH₂-CH₂- | -CH₂-CH₂-CH₂-NH₂ | -CH₂-CH(CH₃)-CH₂-NH₂ |
| 19c | -CH₂-CH₂-CH₂- | -CH₂-CH₂-CH₂-NH₂ | -CH₂-CH(CH₃)-CH₂-NH₂ |
| 19d | -CH₂-CH(OH)- | -CH₂-CH₂-CH₂-NH₂ | -CH₂-CH(CH₃)-CH₂-NH₂ |
| 19e | -CH(OH)-CH₂- | -CH₂-CH₂-CH₂-NH₂ | -CH₂-CH(CH₃)-CH₂-NH₂ |
| 19f | -CH₂-CH(CH₃)- | -CH₂-CH₂-CH₂-NH₂ | -CH₂-CH(CH₃)-CH₂-NH₂ |
| 19g | -CH(CH₃)-CH₂- | -CH₂-CH₂-CH₂-NH₂ | -CH₂-CH(CH₃)-CH₂-NH₂ |
| 19h | -CH(CH₃)-CH(OH)- | -CH₂-CH₂-CH₂-NH₂ | -CH₂-CH(CH₃)-CH₂-NH₂ |
| 19i | -CH(OH)-CH(CH₃)- | -CH₂-CH₂-CH₂-NH₂ | -CH₂-CH(CH₃)-CH₂-NH₂ |
| 19j | -CH₂-O-CH₂- | -CH₂-CH₂-CH₂-NH₂ | -CH₂-CH(CH₃)-CH₂-NH₂ |
| 19k | -O-CH₂- | -CH₂-CH₂-CH₂-NH₂ | -CH₂-CH(CH₃)-CH₂-NH₂ |
| 19l | -O-CH₂-CH₂- | -CH₂-CH₂-CH₂-NH₂ | -CH₂-CH(CH₃)-CH₂-NH₂ |
| 19m | -CH₂-S-CH₂- | -CH₂-CH₂-CH₂-NH₂ | -CH₂-CH(CH₃)-CH₂-NH₂ |
| 19n | -S-CH₂- | -CH₂-CH₂-CH₂-NH₂ | -CH₂-CH(CH₃)-CH₂-NH₂ |
| 19o | -CH(CO₂H)- | -CH₂-CH₂-CH₂-NH₂ | -CH₂-CH(CH₃)-CH₂-NH₂ |
| 19p | -CH(CO₂Me)- | -CH₂-CH₂-CH₂-NH₂ | -CH₂-CH(CH₃)-CH₂-NH₂ |
| 19q | -CH₂-CH(CO₂H)- | -CH₂-CH₂-CH₂-NH₂ | -CH₂-CH(CH₃)-CH₂-NH₂ |
| 19r | -CH(OH)-CH(CO₂H)- | -CH₂-CH₂-CH₂-NH₂ | -CH₂-CH(CH₃)-CH₂-NH₂ |
| 20a | -CH₂- | -CH₂-CH(CH₃)-CH₂-NH₂ | -CH₂-CH₂-CH₂-NH₂ |
| 20b | -CH₂-CH₂- | -CH₂-CH(CH₃)-CH₂-NH₂ | -CH₂-CH₂-CH₂-NH₂ |
| 20c | -CH₂-CH₂-CH₂- | -CH₂-CH(CH₃)-CH₂-NH₂ | -CH₂-CH₂-CH₂-NH₂ |
| 20d | -CH₂-CH(OH)- | -CH₂-CH(CH₃)-CH₂-NH₂ | -CH₂-CH₂-CH₂-NH₂ |
| 20e | -CH(OH)-CH₂- | -CH₂-CH(CH₃)-CH₂-NH₂ | -CH₂-CH₂-CH₂-NH₂ |
| 20f | -CH₂-CH(CH₃)- | -CH₂-CH(CH₃)-CH₂-NH₂ | -CH₂-CH₂-CH₂-NH₂ |
| 20g | -CH(CH₃)-CH₂- | -CH₂-CH(CH₃)-CH₂-NH₂ | -CH₂-CH₂-CH₂-NH₂ |
| 20h | -CH(CH₃)-CH(OH)- | -CH₂-CH(CH₃)-CH₂-NH₂ | -CH₂-CH₂-CH₂-NH₂ |
| 20i | -CH(OH)-CH(CH₃)- | -CH₂-CH(CH₃)-CH₂-NH₂ | -CH₂-CH₂-CH₂-NH₂ |
| 20j | -CH₂-O-CH₂- | -CH₂-CH(CH₃)-CH₂-NH₂ | -CH₂-CH₂-CH₂-NH₂ |
| 20k | -O-CH2- | -CH₂-CH(CH₃)-CH₂-NH₂ | -CH₂-CH₂-CH₂-NH₂ |
| 20l | -O-CH₂-CH₂- | -CH₂-CH(CH₃)-CH₂-NH₂ | -CH₂-CH₂-CH₂-NH₂ |
| 20m | -CH₂-S-CH₂- | -CH₂-CH(CH₃)-CH₂-NH₂ | -CH₂-CH₂-CH₂-NH₂ |
| 20n | -S-CH₂- | -CH₂-CH(CH₃)-CH₂-NH₂ | -CH₂-CH₂-CH₂-NH₂ |
| 20o | -CH(CO₂H)- | -CH₂-CH(CH₃)-CH₂-NH₂ | -CH₂-CH₂-CH₂-NH₂ |
| 20p | -CH(CO₂Me)- | -CH₂-CH(CH₃)-CH₂-NH₂ | -CH₂-CH₂-CH₂-NH₂ |
| 20q | -CH₂-CH(CO₂H)- | -CH₂-CH(CH₃)-CH₂-NH₂ | -CH₂-CH₂-CH₂-NH₂ |
| 20r | -CH(OH)-CH(CO₂H)- | -CH₂-CH(CH₃)-CH₂-NH₂ | -CH₂-CH₂-CH₂-NH₂ |
| 21a | -CH₂- | -CH₂-CH(CH₃)-CH₂-NH₂ | -CH₂-CH(CH₃)-CH₂-NH₂ |
| 21 b | -CH₂-CH₂- | -CH₂-CH(CH₃)-CH₂-NH₂ | -CH₂-CH(CH₃)-CH₂-NH₂ |
| 21c | -CH₂-CH₂-CH₂- | -CH₂-CH(CH₃)-CH₂-NH₂ | -CH₂-CH(CH₃)-CH₂-NH₂ |
| 21d | -CH₂-CH(OH)- | -CH₂-CH(CH₃)-CH₂-NH₂ | -CH₂-CH(CH₃)-CH₂-NH₂ |
| 21e | -CH(OH)-CH₂- | -CH₂-CH(CH₃)-CH₂-NH₂ | -CH₂-CH(CH₃)-CH₂-NH₂ |
| 21f | -CH₂-CH(CH₃)- | -CH₂-CH(CH₃)-CH₂-NH₂ | -CH₂-CH(CH₃)-CH₂-NH₂ |
| 21g | -CH(CH₃)-CH₂- | -CH₂-CH(CH₃)-CH₂-NH₂ | -CH₂-CH(CH₃)-CH₂-NH₂ |
| 21h | -CH(CH₃)-CH(OH)- | -CH₂-CH(CH₃)-CH₂-NH₂ | -CH₂-CH(CH₃)-CH₂-NH₂ |
| 21 i | -CH(OH)-CH(CH₃)- | -CH₂-CH(CH₃)-CH₂-NH₂ | -CH₂-CH(CH₃)-CH₂-NH₂ |
| 21j | -CH₂-O-CH₂- | -CH₂-CH(CH₃)-CH₂-NH₂ | -CH₂-CH(CH₃)-CH₂-NH₂ |
| 21k | -O-CH₂- | -CH₂-CH(CH₃)-CH₂-NH₂ | -CH₂-CH(CH₃)-CH₂-NH₂ |
| 21l | -O-CH₂-CH₂- | -CH₂-CH(CH₃)-CH₂-NH₂ | -CH₂-CH(CH₃)-CH₂-NH₂ |
| 21m | -CH₂-S-CH₂- | -CH₂-CH(CH₃)-CH₂-NH₂ | -CH₂-CH(CH₃)-CH₂-NH₂ |
| 21n | -S-CH₂- | -CH₂-CH(CH₃)-CH₂-NH₂ | -CH₂-CH(CH₃)-CH₂-NH₂ |
| 210 | -CH(CO₂H)- | -CH₂-CH(CH₃)-CH₂-NH₂ | -CH₂-CH(CH₃)-CH₂-NH₂ |
| 21 p | -CH(CO₂Me)- | -CH₂-CH(CH₃)-CH₂-NH₂ | -CH₂-CH(CH₃)-CH₂-NH₂ |
| 21q | -CH₂-CH(CO₂H)- | -CH₂-CH(CH₃)-CH₂-NH₂ | -CH₂-CH(CH₃)-CH₂-NH₂ |
| 21 r | -CH(OH)-CH(CO₂H)- | -CH₂-CH(CH₃)-CH₂-NH₂ | -CH₂-CH(CH₃)-CH₂-NH₂ |
| 22a | -CH₂- | H | -CH₂-CH₂-NH₂ |
| 22b | -CH₂-CH₂- | H | -CH₂-CH₂-NH₂ |
| 122c | -CH₂-CH₂-CH₂- | H | -CH₂-CH₂-NH₂ |
| 22d | -CH₂-CH(OH)- | H | -CH₂-CH₂-NH₂ |
| 22e | -CH(OH)-CH₂- | H | -CH₂-CH₂-NH₂ |
| 22f | -CH₂-CH(CH₃)- | H | -CH₂-CH₂-NH₂ |
| 22g | -CH(CH₃)-CH₂- | H | -CH₂-CH₂-NH₂ |
| 22h | -CH(CH₃)-CH(OH)- | H | -CH₂-CH₂-NH₂ |
| 22i | -CH(OH)-CH(CH₃)- | H | -CH₂-CH₂-NH₂ |
| 22j | -CH₂-O-CH₂- | H | -CH₂-CH₂-NH₂ |
| 22k | -O-CH₂- | H | -CH₂-CH₂-NH₂ |
| 22l | -O-CH₂-CH₂- | H | -CH₂-CH₂-NH₂ |
| 22m | -CH₂-S-CH₂- | H | -CH₂-CH₂-NH₂ |
| 22n | -S-CH₂- | H | -CH₂-CH₂-NH₂ |
| 22o | -CH(CO₂H)- | H | -CH₂-CH₂-NH₂ |
| 22p | -CH(CO₂Me)- | H | -CH₂-CH₂-NH₂ |
| 22q | -CH₂-CH(CO₂H)- | H | -CH₂-CH₂-NH₂ |
| 22r | -CH(OH)-CH(CO₂H)- | H | -CH₂-CH₂-NH₂ |
| 23a | -CH₂- | H | -CH₂-CH₂-CH₂-NH₂ |
| 23b | -CH₂-CH₂- | H | -CH₂-CH₂-CH₂-NH₂ |
| 23c | -CH₂-CH₂-CH₂- | H | -CH₂-CH₂-CH₂-NH₂ |
| 23d | -CH₂-CH(OH)- | H | -CH₂-CH₂-CH₂-NH₂ |
| 23e | -CH(OH)-CH₂- | H | -CH₂-CH₂-CH₂-NH₂ |
| 23f | -CH₂-CH(CH₃)- | H | -CH₂-CH₂-CH₂-NH₂ |
| 23g | -CH(CH₃)-CH₂- | H | -CH₂-CH₂-CH₂-NH₂ |
| 23h | -CH(CH₃)-CH(OH)- | H | -CH₂-CH₂-CH₂-NH₂ |
| 23i | -CH(OH)-CH(CH₃)- | H | -CH₂-CH₂-CH₂-NH₂ |
| 23j | -CH₂-O-CH₂- | H | -CH₂-CH₂-CH₂-NH₂ |
| 23k | -O-CH₂- | H | -CH₂-CH₂-CH₂-NH₂ |
| 23l | -O-CH₂-CH₂- | H | -CH₂-CH₂-CH₂-NH₂ |
| 23m | -CH₂-S-CH₂- | H | -CH₂-CH₂-CH₂-NH₂ |
| 23n | -S-CH₂- | H | -CH₂-CH₂-CH₂-NH₂ |
| 23o | -CH(CO₂H)- | H | -CH₂-CH₂-CH₂-NH₂ |
| 23p | -CH(CO₂Me)- | H | -CH₂-CH₂-CH₂-NH₂ |
| 23q | -CH₂-CH(CO₂H)- | H | -CH₂-CH₂-CH₂-NH₂ |
| 23r | -CH(OH)-CH(CO₂H)- | H | -CH₂-CH₂-CH₂-NH₂ |
| 24a | -CH₂- | H | -CH₂-CH(CH₃)-CH₂-NH₂ |
| 24b | -CH₂-CH₂- | H | -CH₂-CH(CH₃)-CH₂-NH₂ |
| 24c | -CH₂-CH₂-CH₂- | H | -CH₂-CH(CH₃)-CH₂-NH₂ |
| 24d | -CH₂-CH(OH)- | H | -CH₂-CH(CH₃)-CH₂-NH₂ |
| 24e | -CH(OH)-CH₂- | H | -CH₂-CH(CH₃)-CH₂-NH₂ |
| 24f | -CH₂-CH(CH₃)- | H | -CH₂-CH(CH₃)-CH₂-NH₂ |
| 24g | -CH(CH₃)-CH₂- | H | -CH₂-CH(CH₃)-CH₂-NH₂ |
| 24h | -CH(CH₃)-CH(OH)- | H | -CH₂-CH(CH₃)-CH₂-NH₂ |
| 24i | -CH(OH)-CH(CH₃)- | H | -CH₂-CH(CH₃)-CH₂-NH₂ |
| 24j | -CH₂-O-CH₂- | H | -CH₂-CH(CH₃)-CH₂-NH₂ |
| 24k | -O-CH₂- | H | -CH₂-CH(CH₃)-CH₂-NH₂ |
| 24l | -O-CH₂-CH₂- | H | -CH₂-CH(CH₃)-CH₂-NH₂ |
| 24m | -CH₂-S-CH₂- | H | -CH₂-CH(CH₃)-CH₂-NH₂ |
| 24n | -S-CH₂- | H | -CH₂-CH(CH₃)-CH₂-NH₂ |
| 24o | -CH(CO₂H)- | H | -CH₂-CH(CH₃)-CH₂-NH₂ |
| 24p | -CH(CO₂Me)- | H | -CH₂-CH(CH₃)-CH₂-NH₂ |
| 24q | -CH₂-CH(CO₂H)- | H | -CH₂-CH(CH₃)-CH₂-NH₂ |
| 24r | -CH(OH)-CH(CO₂H)- | H | -CH₂-CH(CH₃)-CH₂-NH₂ |

### Example 4: Examples of compounds obtainable by a method according to the invention are listed hereunder.

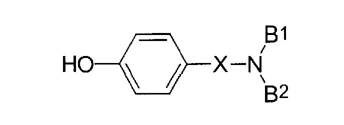

| **Nber** | **X** | **B**^{**1**} | **B**^{**2**} |
|---|---|---|---|
| 25a | -CH₂- | -CH₂-CH₂-NH(CH₃) | -CH₂-CH₂-NH(CH₃) |
| 25b | -CH₂-CH₂- | -CH₂-CH₂-NH(CH₃) | -CH₂-CH₂-NH(CH₃) |
| 25c | -CH₂-CH₂-CH₂- | -CH₂-CH₂-NH(CH₃) | -CH₂-CH₂-NH(CH₃) |
| 25d | -CH₂-CH(OH)- | -CH₂-CH₂-NH(CH₃) | -CH₂-CH₂-NH(CH₃) |
| 25e | -CH(OH)-CH₂- | -CH₂-CH₂-NH(CH₃) | -CH₂-CH₂-NH(CH₃) |
| 25f | -CH₂-CH(CH₃)- | -CH₂-CH₂-NH(CH₃) | -CH₂-CH₂-NH(CH₃) |
| 25g | -CH(CH₃)-CH₂- | -CH₂-CH₂-NH(CH₃) | -CH₂-CH₂-NH(CH₃) |
| 25h | -CH(CH₃)-CH(OH)- | -CH₂-CH₂-NH(CH₃) | -CH₂-CH₂-NH(CH₃) |
| 25i | -CH(OH)-CH(CH₃)- | -CH₂-CH₂-NH(CH₃) | -CH₂-CH₂-NH(CH₃) |
| 25j | -CH₂-O-CH₂- | -CH₂-CH₂-NH(CH₃) | -CH₂-CH₂-NH(CH₃) |
| 25k | -O-CH₂- | -CH₂-CH₂-NH(CH₃) | -CH₂-CH₂-NH(CH₃) |
| 25l | -O-CH₂-CH₂- | -CH₂-CH₂-NH(CH₃) | -CH₂-CH₂-NH(CH₃) |
| 25m | -CH₂-S-CH₂- | -CH₂-CH₂-NH(CH₃) | -CH₂-CH₂-NH(CH₃) |
| 25n | -S-CH₂- | -CH₂-CH₂-NH(CH₃) | -CH₂-CH₂-NH(CH₃) |
| 25o | -CH(CO₂H)- | -CH₂-CH₂-NH(CH₃) | -CH₂-CH₂-NH(CH₃) |
| 25p | -CH(CO₂Me)- | -CH₂-CH₂-NH(CH₃) | -CH₂-CH₂-NH(CH₃) |
| 25q | -CH₂-CH(CO₂H)- | -CH₂-CH₂-NH(CH₃) | -CH₂-CH₂-NH(CH₃) |
| 25r | -CH(OH)CH(CO₂H)- | -CH₂-CH₂-NH(CH₃) | -CH₂-CH₂-NH(CH₃) |
| 26a | -CH₂- | -CH₂-CH₂-CH₂-NH(CH₃) | -CH₂-CH(CH₃)-CH₂-NH₂ |
| 26b | -CH₂-CH₂- | -CH₂-CH₂-CH₂-NH(CH₃) | -CH₂-CH(CH₃)-CH₂-NH₂ |
| 26c | -CH₂-CH₂-CH₂- | -CH₂-CH₂-CH₂-NH(CH₃) | -CH₂-CH(CH₃)-CH₂-NH₂ |
| 26d | -CH₂-CH(OH)- | -CH₂-CH₂-CH₂-NH(CH₃) | -CH₂-CH(CH₃)-CH₂-NH₂ |
| 26e | -CH(OH)-CH₂- | -CH₂-CH₂-CH₂-NH(CH₃) | -CH₂-CH(CH₃)-CH₂-NH₂ |
| 26f | -CH₂-CH(CH₃)- | -CH₂-CH₂-CH₂-NH(CH₃) | -CH₂-CH(CH₃)-CH₂-NH₂ |
| 26g | -CH(CH₃)-CH₂- | -CH₂-CH₂-CH₂-NH(CH₃) | -CH₂-CH(CH₃)-CH₂-NH₂ |
| 26h | -CH(CH₃)-CH(OH)- | -CH₂-CH₂-CH₂-NH(CH₃) | -CH₂-CH(CH₃)-CH₂-NH₂ |
| 26i | -CH(OH)-CH(CH₃)- | -CH₂-CH₂-CH₂-NH(CH₃) | -CH₂-CH(CH₃)-CH₂-NH₂ |
| 26j | -CH₂-O-CH₂- | -CH₂-CH₂-CH₂-NH(CH₃) | -CH₂-CH(CH₃)-CH₂-NH₂ |
| 26k | -O-CH₂- | -CH₂-CH₂-CH₂-NH(CH₃) | -CH₂-CH(CH₃)-CH₂-NH₂ |
| 26l | -O-CH₂-CH₂- | -CH₂-CH₂-CH₂-NH(CH₃) | -CH₂-CH(CH₃)-CH₂-NH₂ |
| 26m | -CH₂-S-CH₂- | -CH₂-CH₂-CH₂-NH(CH₃) | -CH₂-CH(CH₃)-CH₂-NH₂ |
| 26n | -S-CH₂- | -CH₂-CH₂-CH₂-NH(CH₃) | -CH₂-CH(CH₃)-CH₂-NH₂ |
| 260 | -CH(CO₂H)- | -CH₂-CH₂-CH₂-NH(CH₃) | -CH₂-CH(CH₃)-CH₂-NH₂ |
| 26p | -CH(CO₂Me)- | -CH₂-CH₂-CH₂-NH(CH₃) | -CH₂-CH(CH₃)-CH₂-NH₂ |
| 26q | -CH₂-CH(CO₂H)- | -CH₂-CH₂-CH₂-NH(CH₃) | -CH₂-CH(CH₃)-CH₂-NH₂ |
| 26r | -CH(OH)-CH(CO₂H)- | -CH₂-CH₂-CH₂-NH(CH₃) | -CH₂-CH(CH₃)-CH₂-NH₂ |
| 26a | -CH₂- | -CH₂-CH₂-CH₂-NH(CH₃) | -CH₂-CH(CH₃)-CH₂-NH(CH₃) |
| 26b | -CH₂-CH₂- | -CH₂-CH₂-CH₂-NH(CH₃) | -CH₂-CH(CH₃)-CH₂-NH(CH₃) |
| 26c | -CH₂-CH₂-CH₂- | -CH₂-CH₂-CH₂-NH(CH₃) | -CH₂-CH(CH₃)-CH₂-NH(CH₃) |
| 26d | -CH₂-CH(OH)- | -CH₂-CH₂-CH₂-NH(CH₃) | -CH₂-CH(CH₃)-CH₂-NH(CH₃) |
| 26e | -CH(OH)-CH₂- | -CH₂-CH₂-CH₂-NH(CH₃) | -CH₂-CH(CH₃)-CH₂-NH(CH₃) |
| 26f | -CH₂-CH(CH₃)- | -CH₂-CH₂-CH₂-NH(CH₃) | -CH₂-CH(CH₃)-CH₂-NH(CH₃) |
| 26g | -CH(CH₃)-CH₂- | -CH₂-CH₂-CH₂-NH(CH₃) | -CH₂-CH(CH₃)-CH₂-NH(CH₃) |
| 26h | -CH(CH₃)-CH(OH)- | -CH₂-CH₂-CH₂-NH(CH₃) | -CH₂-CH(CH₃)-CH₂-NH(CH₃) |
| 26i | -CH(OH)-CH(CH₃)- | -CH₂-CH₂-CH₂-NH(CH₃) | -CH₂-CH(CH₃)-CH₂-NH(CH₃) |
| 26j | -CH₂-O-CH₂- | -CH₂-CH₂-CH₂-NH(CH₃) | -CH₂-CH(CH₃)-CH₂-NH(CH₃) |
| 26k | -O-CH₂- | -CH₂-CH₂-CH₂-NH(CH₃) | -CH₂-CH(CH₃)-CH₂-NH(CH₃) |
| 26l | -O-CH₂-CH₂- | -CH₂-CH₂-CH₂-NH(CH₃) | -CH₂-CH(CH₃)-CH₂-NH(CH₃) |
| 26m | -CH₂-S-CH₂- | -CH₂-CH₂-CH₂-NH(CH₃) | -CH₂-CH(CH₃)-CH₂-NH(CH₃) |
| 26n | -S-CH₂- | -CH₂-CH₂-CH₂-NH(CH₃) | -CH₂-CH(CH₃)-CH₂-NH(CH₃) |
| 26o | -CH(CO₂H)- | -CH₂-CH₂-CH₂-NH(CH₃) | -CH₂-CH(CH₃)-CH₂-NH(CH₃) |
| 26p | -CH(CO₂Me)- | -CH₂-CH₂-CH₂-NH(CH₃) | -CH₂-CH(CH₃)-CH₂-NH(CH₃) |
| 26q | -CH₂-CH(CO₂H)- | -CH₂-CH₂-CH₂-NH(CH₃) | -CH₂-CH(CH₃)-CH₂-NH(CH₃) |
| 26r | -CH(OH)CH(CO₂H)- | -CH₂-CH₂-CH₂-NH(CH₃) | -CH₂-CH(CH₃)-CH₂-NH(CH₃) |
| 27a | -CH₂- | H | -CH₂-CH₂-NH(CH₃) |
| 27b | -CH₂-CH₂- | H | -CH₂-CH₂-NH(CH₃) |
| 27c | -CH₂-CH₂-CH₂- | H | -CH₂-CH₂-NH(CH₃) |
| 27d | -CH₂-CH(OH)- | H | -CH₂-CH₂-NH(CH₃) |
| 27e | -CH(OH)-CH₂- | H | -CH₂-CH₂-NH(CH₃) |
| 27f | -CH₂-CH(CH₃)- | H | -CH₂-CH₂-NH(CH₃) |
| 27g | -CH(CH₃)-CH₂- | H | -CH₂-CH₂-NH(CH₃) |
| 27h | -CH(CH₃)-CH(OH)- | H | -CH₂-CH₂-NH(CH₃) |
| 27i | -CH(OH)-CH(CH₃)- | H | -CH₂-CH₂-NH(CH₃) |
| 27j | -CH₂-O-CH₂- | H | -CH₂-CH₂-NH(CH₃) |
| 27k | -O-CH₂- | H | -CH₂-CH₂-NH(CH₃) |
| 27l | -O-CH₂-CH₂- | H | -CH₂-CH₂-NH(CH₃) |
| 27m | -CH₂-S-CH₂- | H | -CH₂-CH₂-NH(CH₃) |
| 27n | -S-CH₂- | H | -CH₂-CH₂-NH(CH₃) |
| 27o | -CH(CO₂H)- | H | -CH₂-CH₂-NH(CH₃) |
| 27p | -CH(CO₂Me)- | H | -CH₂-CH₂-NH(CH₃) |
| 27q | -CH₂-CH(CO₂H)- | H | -CH₂-CH₂-NH(CH₃) |
| 27r | -CH(OH)CH(CO₂H)- | H | -CH₂-CH₂-NH(CH₃) |
| 28a | -CH₂- | H | -CH₂-CH(CH₃)-CH₂-NH(CH₃) |
| 278b | -CH₂-CH₂- | H | -CH₂-CH(CH₃)-CH₂-NH(CH₃) |
| 28c | -CH₂-CH₂-CH₂- | H | -CH₂-CH(CH₃)-CH₂-NH(CH₃) |
| 28d | -CH₂-CH(OH)- | H | -CH₂-CH(CH₃)-CH₂-NH(CH₃) |
| 28e | -CH(OH)-CH₂- | H | -CH₂-CH(CH₃)-CH₂-NH(CH₃) |
| 28f | -CH₂-CH(CH₃)- | H | -CH₂-CH(CH₃)-CH₂-NH(CH₃) |
| 28g | -CH(CH₃)-CH₂- | H | -CH₂-CH(CH₃)-CH₂-NH(CH₃) |
| 208h | -CH(CH₃)-CH(OH)- | H | -CH₂-CH(CH₃)-CH₂-NH(CH₃) |
| 28i | -CH(OH)-CH(CH₃)- | H | -CH₂-CH(CH₃)-CH₂-NH(CH₃) |
| 28j | -CH₂-O-CH₂- | H | -CH₂-CH(CH₃)-CH₂-NH(CH₃) |
| 28k | -O-CH₂- | H | -CH₂-CH(CH₃)-CH₂-NH(CH₃) |
| 28l | -O-CH₂-CH₂- | H | -CH₂-CH(CH₃)-CH₂-NH(CH₃) |
| 28m | -CH₂-S-CH₂- | H | -CH₂-CH(CH₃)-CH₂-NH(CH₃) |
| 28n | -S-CH₂- | H | -CH₂-CH(CH₃)-CH₂-NH(CH₃) |
| 28o | -CH(CO₂H)- | H | -CH₂-CH(CH₃)-CH₂-NH(CH₃) |
| 28p | -CH(CO₂Me)- | H | -CH₂-CH(CH₃)-CH₂-NH(CH₃) |
| 28q | -CH₂-CH(CO₂H)- | H | -CH₂-CH(CH₃)-CH₂-NH(CH₃) |
| 28r | -CH(OH)-CH(CO₂H)- | H | -CH₂-CH(CH₃)-CH₂-NH(CH₃) |

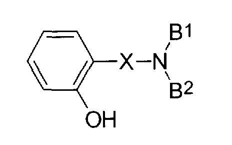

| **Nber** | **X** | **B**^{**1**} | **B**^{**2**} |
|---|---|---|---|
| 29a | -CH₂- | -CH₂-CH₂-NH(CH₃) | -CH₂-CH₂-NH(CH₃) |
| 29b | -CH₂-CH₂- | -CH₂-CH₂-NH(CH₃) | -CH₂-CH₂-NH(CH₃) |
| 29c | -CH₂-CH₂-CH₂- | -CH₂-CH₂-NH(CH₃) | -CH₂-CH₂-NH(CH₃) |
| 29d | -CH₂-CH(OH)- | -CH₂-CH₂-NH(CH₃) | -CH₂-CH₂-NH(CH₃) |
| 29e | -CH(OH)-CH₂- | -CH₂-CH₂-NH(CH₃) | -CH₂-CH₂-NH(CH₃) |
| 29f | -CH₂-CH(CH₃)- | -CH₂-CH₂-NH(CH₃) | -CH₂-CH₂-NH(CH₃) |
| 29g | -CH(CH₃)-CH₂- | -CH₂-CH₂-NH(CH₃) | -CH₂-CH₂-NH(CH₃) |
| 29h | -CH(CH₃)-CH(OH)- | -CH₂-CH₂-NH(CH₃) | -CH₂-CH₂-NH(CH₃) |
| 29i | -CH(OH)-CH(CH₃)- | -CH₂-CH₂-NH(CH₃) | -CH₂-CH₂-NH(CH₃) |
| 29j | -CH₂-O-CH₂- | -CH₂-CH₂-NH(CH₃) | -CH₂-CH₂-NH(CH₃) |
| 29k | -O-CH₂- | -CH₂-CH₂-NH(CH₃) | -CH₂-CH₂-NH(CH₃) |
| 29l | -O-CH₂-CH₂- | -CH₂-CH₂-NH(CH₃) | -CH₂-CH₂-NH(CH₃) |
| 29m | -CH₂-S-CH₂- | -CH₂-CH₂-NH(CH₃) | -CH₂-CH₂-NH(CH₃) |
| 29n | -S-CH₂- | -CH₂-CH₂-NH(CH₃) | -CH₂-CH₂-NH(CH₃) |
| 29o | -CH(CO₂H)- | -CH₂-CH₂-NH(CH₃) | -CH₂-CH₂-NH(CH₃) |
| 29p | -CH(CO₂Me)- | -CH₂-CH₂-NH(CH₃) | -CH₂-CH₂-NH(CH₃) |
| 29q | -CH₂-CH(CO₂H)- | -CH₂-CH₂-NH(CH₃) | -CH₂-CH₂-NH(CH₃) |
| 29r | -CH(OH)-CH(CO₂H)- | -CH₂-CH₂-NH(CH₃) | -CH₂-CH₂-NH(CH₃) |
| 30a | -CH₂- | -CH₂-CH₂-CH₂-NH(CH₃) | -CH₂-CH(CH₃)-CH₂-NH₂ |
| 30b | -CH₂-CH₂- | -CH₂-CH₂-CH₂-NH(CH₃) | -CH₂-CH(CH₃)-CH₂-NH₂ |
| 30c | -CH₂-CH₂-CH₂- | -CH₂-CH₂-CH₂-NH(CH₃) | -CH₂-CH(CH₃)-CH₂-NH₂ |
| 30d | -CH₂-CH(OH)- | -CH₂-CH₂-CH₂-NH(CH₃) | -CH₂-CH(CH₃)-CH₂-NH₂ |
| 30e | -CH(OH)-CH₂- | -CH₂-CH₂-CH₂-NH(CH₃) | -CH₂-CH(CH₃)-CH₂-NH₂ |
| 30f | -CH₂-CH(CH₃)- | -CH₂-CH₂-CH₂-NH(CH₃) | -CH₂-CH(CH₃)-CH₂-NH₂ |
| 30g | -CH(CH₃)-CH₂- | -CH₂-CH₂-CH₂-NH(CH₃) | -CH₂-CH(CH₃)-CH₂-NH₂ |
| 30h | -CH(CH₃)-CH(OH)- | -CH₂-CH₂-CH₂-NH(CH₃) | -CH₂-CH(CH₃)-CH₂-NH₂ |
| 30i | -CH(OH)-CH(CH₃)- | -CH₂-CH₂-CH₂-NH(CH₃) | -CH₂-CH(CH₃)-CH₂-NH₂ |
| 30j | -CH₂-O-CH₂- | -CH₂-CH₂-CH₂-NH(CH₃) | -CH₂-CH(CH₃)-CH₂-NH₂ |
| 30k | -O-CH₂- | -CH₂-CH₂-CH₂-NH(CH₃) | -CH₂-CH(CH₃)-CH₂-NH₂ |
| 30l | -O-CH₂-CH₂- | -CH₂-CH₂-CH₂-NH(CH₃) | -CH₂-CH(CH₃)-CH₂-NH₂ |
| 30m | -CH₂-S-CH₂- | -CH₂-CH₂-CH₂-NH(CH₃) | -CH₂-CH(CH₃)-CH₂-NH₂ |
| 30n | -S-CH₂- | -CH₂-CH₂-CH₂-NH(CH₃) | -CH₂-CH(CH₃)-CH₂-NH₂ |
| 30o | -CH(CO₂H)- | -CH₂-CH₂-CH₂-NH(CH₃) | -CH₂-CH(CH₃)-CH₂-NH₂ |
| 30p | -CH(CO₂Me)- | -CH₂-CH₂-CH₂-NH(CH₃) | -CH₂-CH(CH₃)-CH₂-NH₂ |
| 30q | -CH₂-CH(CO₂H)- | -CH₂-CH₂-CH₂-NH(CH₃) | -CH₂-CH(CH₃)-CH₂-NH₂ |
| 30r | -CH(OH)-CH(CO₂H)- | -CH₂-CH₂-CH₂-NH(CH₃) | -CH₂-CH(CH₃)-CH₂-NH₂ |
| 31a | -CH₂- | -CH₂-CH₂-CH₂-NH(CH₃) | -CH₂-CH(CH₃)-CH₂-NH(CH₃) |
| 31b | -CH₂-CH₂- | -CH₂-CH₂-CH₂-NH(CH₃) | -CH₂-CH(CH₃)-CH₂-NH(CH₃) |
| 31c | -CH₂-CH₂-CH₂- | -CH₂-CH₂-CH₂-NH(CH₃) | -CH₂-CH(CH₃)-CH₂-NH(CH₃) |
| 31d | -CH₂-CH(OH)- | -CH₂-CH₂-CH₂-NH(CH₃) | -CH₂-CH(CH₃)-CH₂-NH(CH₃) |
| 31e | -CH(OH)-CH₂- | -CH₂-CH₂-CH₂-NH(CH₃) | -CH₂-CH(CH₃)-CH₂-NH(CH₃) |
| 31f | -CH₂-CH(CH₃)- | -CH₂-CH₂-CH₂-NH(CH₃) | -CH₂-CH(CH₃)-CH₂-NH(CH₃) |
| 31g | -CH(CH₃)-CH₂- | -CH₂-CH₂-CH₂-NH(CH₃) | -CH₂-CH(CH₃)-CH₂-NH(CH₃) |
| 31h | -CH(CH₃)-CH(OH)- | -CH₂-CH₂-CH₂-NH(CH₃) | -CH₂-CH(CH₃)-CH₂-NH(CH₃) |
| 31 i | -CH(OH)-CH(CH₃)- | -CH₂-CH₂-CH₂-NH(CH₃) | -CH₂-CH(CH₃)-CH₂-NH(CH₃) |
| 31j | -CH₂-O-CH₂- | -CH₂-CH₂-CH₂-NH(CH₃) | -CH₂-CH(CH₃)-CH₂-NH(CH₃) |
| 31k | -O-CH₂- | -CH₂-CH₂-CH₂-NH(CH₃) | -CH₂-CH(CH₃)-CH₂-NH(CH₃) |
| 31l | -O-CH₂-CH₂- | -CH₂-CH₂-CH₂-NH(CH₃) | -CH₂-CH(CH₃)-CH₂-NH(CH₃) |
| 31m | -CH₂-S-CH₂- | -CH₂-CH₂-CH₂-NH(CH₃) | -CH₂-CH(CH₃)-CH₂-NH(CH₃) |
| 31n | -S-CH₂- | -CH₂-CH₂-CH₂-NH(CH₃) | -CH₂-CH(CH₃)-CH₂-NH(CH₃) |
| 310 | -CH(CO₂H)- | -CH₂-CH₂-CH₂-NH(CH₃) | -CH₂-CH(CH₃)-CH₂-NH(CH₃) |
| 31p | -CH(CO₂Me)- | -CH₂-CH₂-CH₂-NH(CH₃) | -CH₂-CH(CH₃)-CH₂-NH(CH₃) |
| 31q | -CH₂-CH(CO₂H)- | -CH₂-CH₂-CH₂-NH(CH₃) | -CH₂-CH(CH₃)-CH₂-NH(CH₃) |
| 31 r | -CH(OH)-CH(CO₂H)- | -CH₂-CH₂-CH₂-NH(CH₃) | -CH₂-CH(CH₃)-CH₂-NH(CH₃) |
| 32a | -CH₂- | H | -CH₂-CH₂-NH(CH₃) |
| 32b | -CH₂-CH₂- | H | -CH₂-CH₂-NH(CH₃) |
| 32c | -CH₂-CH₂-CH₂- | H | -CH₂-CH₂-NH(CH₃) |
| 32d | -CH₂-CH(OH)- | H | -CH₂-CH₂-NH(CH₃) |
| 32e | -CH(OH)-CH₂- | H | -CH₂-CH₂-NH(CH₃) |
| 32f | -CH₂-CH(CH₃)- | H | -CH₂-CH₂-NH(CH₃) |
| 32g | -CH(CH₃)-CH₂- | H | -CH₂-CH₂-NH(CH₃) |
| 32h | -CH(CH₃)-CH(OH)- | H | -CH₂-CH₂-NH(CH₃) |
| 32i | -CH(OH)-CH(CH₃)- | H | -CH₂-CH₂-NH(CH₃) |
| 32j | -CH₂-O-CH₂- | H | -CH₂-CH₂-NH(CH₃) |
| 32k | -O-CH₂- | H | -CH₂-CH₂-NH(CH₃) |
| 32l | -O-CH₂-CH₂- | H | -CH₂-CH₂-NH(CH₃) |
| 32m | -CH₂-S-CH₂- | H | -CH₂-CH₂-NH(CH₃) |
| 32n | -S-CH₂- | H | -CH₂-CH₂-NH(CH₃) |
| 32o | -CH(CO₂H)- | H | -CH₂-CH₂-NH(CH₃) |
| 32p | -CH(CO₂Me)- | H | -CH₂-CH₂-NH(CH₃) |
| 32q | -CH₂-CH(CO₂H)- | H | -CH₂-CH₂-NH(CH₃) |
| 32r | -CH(OH)-CH(CO₂H)- | H | -CH₂-CH₂-NH(CH₃) |
| 33a | -CH₂- | H | -CH₂-CH(CH₃)-CH₂-NH(CH₃) |
| 33b | -CH₂-CH₂- | H | -CH₂-CH(CH₃)-CH₂-NH(CH₃) |
| 33c | -CH₂-CH₂-CH₂- | H | -CH₂-CH(CH₃)-CH₂-NH(CH₃) |
| 33d | -CH₂-CH(OH)- | H | -CH₂-CH(CH₃)-CH₂-NH(CH₃) |
| 33e | -CH(OH)-CH₂- | H | -CH₂-CH(CH₃)-CH₂-NH(CH₃) |
| 33f | -CH₂-CH(CH₃)- | H | -CH₂-CH(CH₃)-CH₂-NH(CH₃) |
| 33g | -CH(CH₃)-CH₂- | H | -CH₂-CH(CH₃)-CH₂-NH(CH₃) |
| 33h | -CH(CH₃)-CH(OH)- | H | -CH₂-CH(CH₃)-CH₂-NH(CH₃) |
| 33i | -CH(OH)-CH(CH₃)- | H | -CH₂-CH(CH₃)-CH₂-NH(CH₃) |
| 33j | -CH₂-O-CH₂- | H | -CH₂-CH(CH₃)-CH₂-NH(CH₃) |
| 33k | -O-CH₂- | H | -CH₂-CH(CH₃)-CH₂-NH(CH₃) |
| 33 | -O-CH₂-CH₂- | H | -CH₂-CH(CH₃)-CH₂-NH(CH₃) |
| 33m | -CH₂-S-CH₂- | H | -CH₂-CH(CH₃)-CH₂-NH(CH₃) |
| 33n | -S-CH₂- | H | -CH₂-CH(CH₃)-CH₂-NH(CH₃) |
| 33o | -CH(CO₂H)- | H | -CH₂-CH(CH₃)-CH₂-NH(CH₃) |
| 33p | -CH(CO₂Me)- | H | -CH₂-CH(CH₃)-CH₂-NH(CH₃) |
| 33q | -CH₂-CH(CO₂H)- | H | -CH₂-CH(CH₃)-CH₂-NH(CH₃) |
| 33r | -CH(OH)-CH(CO₂H)- | H | -CH₂-CH(CH₃)-CH₂-NH(CH₃) |

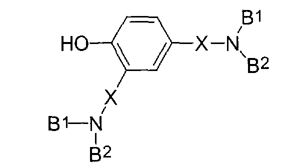

| **Nber** | **X** | **B**^{**1**} | **B**^{**2**} |
|---|---|---|---|
| 34a | -CH₂- | -CH₂-CH₂-NH(CH₃) | -CH₂-CH₂-NH(CH₃) |
| 34b | -CH₂-CH₂- | -CH₂-CH₂-NH(CH₃) | -CH₂-CH₂-NH(CH₃) |
| 34c | -CH₂-CH₂-CH₂- | -CH₂-CH₂-NH(CH₃) | -CH₂-CH₂-NH(CH₃) |
| 34d | -CH₂-CH(OH)- | -CH₂-CH₂-NH(CH₃) | -CH₂-CH₂-NH(CH₃) |
| 34e | -CH(OH)-CH₂- | -CH₂-CH₂-NH(CH₃) | -CH₂-CH₂-NH(CH₃) |
| 34f | -CH₂-CH(CH₃)- | -CH₂-CH₂-NH(CH₃) | -CH₂-CH₂-NH(CH₃) |
| 34g | -CH(CH₃)-CH₂- | -CH₂-CH₂-NH(CH₃) | -CH₂-CH₂-NH(CH₃) |
| 34h | -CH(CH₃)-CH(OH)- | -CH₂-CH₂-NH(CH₃) | -CH₂-CH₂-NH(CH₃) |
| 34i | -CH(OH)-CH(CH₃)- | -CH₂-CH₂-NH(CH₃) | -CH₂-CH₂-NH(CH₃) |
| 34j | -CH₂-O-CH₂- | -CH₂-CH₂-NH(CH₃) | -CH₂-CH₂-NH(CH₃) |
| 34k | -O-CH₂- | -CH₂-CH₂-NH(CH₃) | -CH₂-CH₂-NH(CH₃) |
| 34l | -O-CH₂-CH₂- | -CH₂-CH₂-NH(CH₃) | -CH₂-CH₂-NH(CH₃) |
| 34m | -CH₂-S-CH₂- | -CH₂-CH₂-NH(CH₃) | -CH₂-CH₂-NH(CH₃) |
| 34n | -S-CH₂- | -CH₂-CH₂-NH(CH₃) | -CH₂-CH₂-NH(CH₃) |
| 34o | -CH(CO₂H)- | -CH₂-CH₂-NH(CH₃) | -CH₂-CH₂-NH(CH₃) |
| 34p | -CH(CO₂Me)- | -CH₂-CH₂-NH(CH₃) | -CH₂-CH₂-NH(CH₃) |
| 34q | -CH₂-CH(CO₂H)- | -CH₂-CH₂-NH(CH₃) | -CH₂-CH₂-NH(CH₃) |
| 34r | -CH(OH)-CH(CO₂H)- | -CH₂-CH₂-NH(CH₃) | -CH₂-CH₂-NH(CH₃) |
| 35a | -CH₂- | -CH₂-CH₂-CH₂-NH(CH₃) | -CH₂-CH(CH₃)-CH₂-NH₂ |
| 35b | -CH₂-CH₂- | -CH₂-CH₂-CH₂-NH(CH₃) | -CH₂-CH(CH₃)-CH₂-NH₂ |
| 35c | -CH₂-CH₂-CH₂- | -CH₂-CH₂-CH₂-NH(CH₃) | -CH₂-CH(CH₃)-CH₂-NH₂ |
| 35d | -CH₂-CH(OH)- | -CH₂-CH₂-CH₂-NH(CH₃) | -CH₂-CH(CH₃)-CH₂-NH₂ |
| 35e | -CH(OH)-CH₂- | -CH₂-CH₂-CH₂-NH(CH₃) | -CH₂-CH(CH₃)-CH₂-NH₂ |
| 35f | -CH₂-CH(CH₃)- | -CH₂-CH₂-CH₂-NH(CH₃) | -CH₂-CH(CH₃)-CH₂-NH₂ |
| 35g | -CH(CH₃)-CH₂- | -CH₂-CH₂-CH₂-NH(CH₃) | -CH₂-CH(CH₃)-CH₂-NH₂ |
| 35h | -CH(CH₃)-CH(OH)- | -CH₂-CH₂-CH₂-NH(CH₃) | -CH₂-CH(CH₃)-CH₂-NH₂ |
| 35i | -CH(OH)-CH(CH₃)- | -CH₂-CH₂-CH₂-NH(CH₃) | -CH₂-CH(CH₃)-CH₂-NH₂ |
| 35j | -CH₂-O-CH₂- | -CH₂-CH₂-CH₂-NH(CH₃) | -CH₂-CH(CH₃)-CH₂-NH₂ |
| 35k | -O-CH₂- | -CH₂-CH₂-CH₂-NH(CH₃) | -CH₂-CH(CH₃)-CH₂-NH₂ |
| 35l | -O-CH₂-CH₂- | -CH₂-CH₂-CH₂-NH(CH₃) | -CH₂-CH(CH₃)-CH₂-NH₂ |
| 35m | -CH₂-S-CH₂- | -CH₂-CH₂-CH₂-NH(CH₃) | -CH₂-CH(CH₃)-CH₂-NH₂ |
| 35n | -S-CH₂- | -CH₂-CH₂-CH₂-NH(CH₃) | -CH₂-CH(CH₃)-CH₂-NH₂ |
| 35o | -CH(CO₂H)- | -CH₂-CH₂-CH₂-NH(CH₃) | -CH₂-CH(CH₃)-CH₂-NH₂ |
| 35p | -CH(CO₂Me)- | -CH₂-CH₂-CH₂-NH(CH₃) | -CH₂-CH(CH₃)-CH₂-NH₂ |
| 35q | -CH₂-CH(CO₂H)- | -CH₂-CH₂-CH₂-NH(CH₃) | -CH₂-CH(CH₃)-CH₂-NH₂ |
| 35r | -CH(OH)-CH(CO₂H)- | -CH₂-CH₂-CH₂-NH(CH₃) | -CH₂-CH(CH₃)-CH₂-NH₂ |
| 36a | -CH₂- | -CH₂-CH₂-CH₂-NH(CH₃) | -CH₂-CH(CH₃)-CH₂-NH(CH₃) |
| 36b | -CH₂-CH₂- | -CH₂-CH₂-CH₂-NH(CH₃) | -CH₂-CH(CH₃)-CH₂-NH(CH₃) |
| 36c | -CH₂-CH₂-CH₂- | -CH₂-CH₂-CH₂-NH(CH₃) | -CH₂-CH(CH₃)-CH₂-NH(CH₃) |
| 36d | -CH₂-CH(OH)- | -CH₂-CH₂-CH₂-NH(CH₃) | -CH₂-CH(CH₃)-CH₂-NH(CH₃) |
| 36e | -CH(OH)-CH₂- | -CH₂-CH₂-CH₂-NH(CH₃) | -CH₂-CH(CH₃)-CH₂-NH(CH₃) |
| 36f | -CH₂-CH(CH₃)- | -CH₂-CH₂-CH₂-NH(CH₃) | -CH₂-CH(CH₃)-CH₂-NH(CH₃) |
| 36g | -CH(CH₃)-CH₂- | -CH₂-CH₂-CH₂-NH(CH₃) | -CH₂-CH(CH₃)-CH₂-NH(CH₃) |
| 36h | -CH(CH₃)-CH(OH)- | -CH₂-CH₂-CH₂-NH(CH₃) | -CH₂-CH(CH₃)-CH₂-NH(CH₃) |
| 36i | -CH(OH)-CH(CH₃)- | -CH₂-CH₂-CH₂-NH(CH₃) | -CH₂-CH(CH₃)-CH₂-NH(CH₃) |
| 36j | -CH₂-O-CH₂- | -CH₂-CH₂-CH₂-NH(CH₃) | -CH₂-CH(CH₃)-CH₂-NH(CH₃) |
| 36k | -O-CH₂- | -CH₂-CH₂-CH₂-NH(CH₃) | -CH₂-CH(CH₃)-CH₂-NH(CH₃) |
| 36l | -O-CH₂-CH₂- | -CH₂-CH₂-CH₂-NH(CH₃) | -CH₂-CH(CH₃)-CH₂-NH(CH₃) |
| 36m | -CH₂-S-CH₂- | -CH₂-CH₂-CH₂-NH(CH₃) | -CH₂-CH(CH₃)-CH₂-NH(CH₃) |
| 36n | -S-CH₂- | -CH₂-CH₂-CH₂-NH(CH₃) | -CH₂-CH(CH₃)-CH₂-NH(CH₃) |
| 36o | -CH(CO₂H)- | -CH₂-CH₂-CH₂-NH(CH₃) | -CH₂-CH(CH₃)-CH₂-NH(CH₃) |
| 36p | -CH(CO₂Me)- | -CH₂-CH₂-CH₂-NH(CH₃) | -CH₂-CH(CH₃)-CH₂-NH(CH₃) |
| 36q | -CH₂-CH(CO₂H)- | -CH₂-CH₂-CH₂-NH(CH₃) | -CH₂-CH(CH₃)-CH₂-NH(CH₃) |
| 36r | -CH(OH)-CH(CO₂H)- | -CH₂-CH₂-CH₂-NH(CH₃) | -CH₂-CH(CH₃)-CH₂-NH(CH₃) |
| 37a | -CH₂- | H | -CH₂-CH₂-NH(CH₃) |
| 37b | -CH₂-CH₂- | H | -CH₂-CH₂-NH(CH₃) |
| 37c | -CH₂-CH₂-CH₂- | H | -CH₂-CH₂-NH(CH₃) |
| 37d | -CH₂-CH(OH)- | H | -CH₂-CH₂-NH(CH₃) |
| 37e | -CH(OH)-CH₂- | H | -CH₂-CH₂-NH(CH₃) |
| 37f | -CH₂-CH(CH₃)- | H | -CH₂-CH₂-NH(CH₃) |
| 37g | -CH(CH₃)-CH₂- | H | -CH₂-CH₂-NH(CH₃) |
| 37h | -CH(CH₃)-CH(OH)- | H | -CH₂-CH₂-NH(CH₃) |
| 37i | -CH(OH)-CH(CH₃)- | H | -CH₂-CH₂-NH(CH₃) |
| 37j | -CH₂-O-CH₂- | H | -CH₂-CH₂-NH(CH₃) |
| 37k | -O-CH₂- | H | -CH₂-CH₂-NH(CH₃) |
| 37l | -O-CH₂-CH₂- | H | -CH₂-CH₂-NH(CH₃) |
| 37m | -CH₂-S-CH₂- | H | -CH₂-CH₂-NH(CH₃) |
| 37n | -S-CH₂- | H | -CH₂-CH₂-NH(CH₃) |
| 37o | -CH(CO₂H)- | H | -CH₂-CH₂-NH(CH₃) |
| 37p | -CH(CO₂Me)- | H | -CH₂-CH₂-NH(CH₃) |
| 37q | -CH₂-CH(CO₂H)- | H | -CH₂-CH₂-NH(CH₃) |
| 37r | -CH(OH)-CH(CO₂H)- | H | -CH₂-CH₂-NH(CH₃) |
| 38a | -CH₂- | H | -CH₂-CH(CH₃)-CH₂-NH(CH₃) |
| 38b | -CH₂-CH₂- | H | -CH₂-CH(CH₃)-CH₂-NH(CH₃) |
| 38c | -CH₂-CH₂-CH₂- | H | -CH₂-CH(CH₃)-CH₂-NH(CH₃) |
| 38d | -CH₂-CH(OH)- | H | -CH₂-CH(CH₃)-CH₂-NH(CH₃) |
| 38e | -CH(OH)-CH₂- | H | -CH₂-CH(CH₃)-CH₂-NH(CH₃) |
| 38f | -CH₂-CH(CH₃)- | H | -CH₂-CH(CH₃)-CH₂-NH(CH₃) |
| 38g | -CH(CH₃)-CH₂- | H | -CH₂-CH(CH₃)-CH₂-NH(CH₃) |
| 38h | -CH(CH₃)-CH(OH)- | H | -CH₂-CH(CH₃)-CH₂-NH(CH₃) |
| 38i | -CH(OH)-CH(CH₃)- | H | -CH₂-CH(CH₃)-CH₂-NH(CH₃) |
| 38j | -CH₂-O-CH₂- | H | -CH₂-CH(CH₃)-CH₂-NH(CH₃) |
| 38k | -O-CH₂- | H | -CH₂-CH(CH₃)-CH₂-NH(CH₃) |
| 38l | -O-CH₂-CH₂- | H | -CH₂-CH(CH₃)-CH₂-NH(CH₃) |
| 38m | -CH₂-S-CH₂- | H | -CH₂-CH(CH₃)-CH₂-NH(CH₃) |
| 38n | -S-CH₂- | H | -CH₂-CH(CH₃)-CH₂-NH(CH₃) |
| 38o | -CH(CO₂H)- | H | -CH₂-CH(CH₃)-CH₂-NH(CH₃) |
| 38p | -CH(CO₂Me)- | H | -CH₂-CH(CH₃)-CH₂-NH(CH₃) |
| 38q | -CH₂-CH(CO₂H)- | H | -CH₂-CH(CH₃)-CH₂-NH(CH₃) |
| 38r | -CH(OH)-CH(CO₂H)- | H | -CH₂-CH(CH₃)-CH₂-NH(CH₃) |

### Example 5: The compounds according to the invention may be also prepared using alternative methods as described hereunder in embodiments illustrated in Scheme 2 and Scheme 3.

Synthetic route as depicted in Scheme 2.

Tyramine is first reacted with aziridine to give N,N-(diaminoethyl)4-(2-aminoethyl)phenol. Said N,N-(diaminoethyl)4-(2-aminoethyl)phenol may be further reacted with acrylonitrile or methacrylonitrile followed by a catalytic hydrogenation to give compound of formula (V) 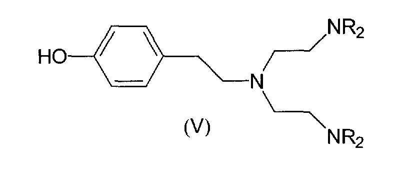 wherein R is H, (CH₂)₃NH₂ or CH₂CH(CH₃)CH₂NH₂. The compound of formula (V) may be further reacted with acrylonitrile or methacrylonitrile followed by a catalytic hydrogenation to give polyaminoalkylated phenolic compounds.

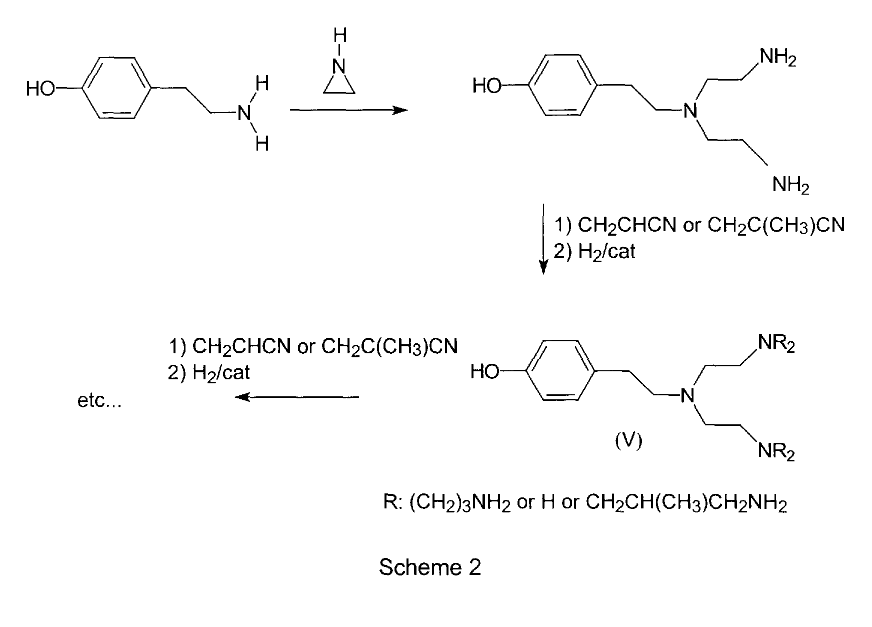

### Synthetic route as depicted in Scheme 3:

Tyramine is first reacted with butadiene to give N,N-(dibut-3-ene)-4-(2-aminoethyl)phenol (**a**). Said N,N-(dibut-3-ene)-4-(2-aminoethyl)phenol (**a**) is further reacted with hydrogen cyanide. According to the site of addition, said reaction can produce 3 different compounds: N,N-(di-4-cyanobutyl)-4-(2-aminoethyl)phenol (**b**), N,N-(di-3-cyanobutyl)-4-(2-aminoethyl)phenol (**c**), (N-4-cyanobutyl)(N-3-cyanobutyl)-4-(2-aminomethyl)phenol (**d**). These compounds are, in a further step, reduced under catalytic hydrogenation to give respectively N,N-(di-aminopentyl)-4-(2-aminoethyl)phenol (**e**), N,N-(di-3(aminomethyl)butyl)-4-(2-aminoethyl)phenol (**f**), (N-aminopentyl)(N-3-aminomethyl)butyl)-4-(2-aminomethyl)phenol (**g**).

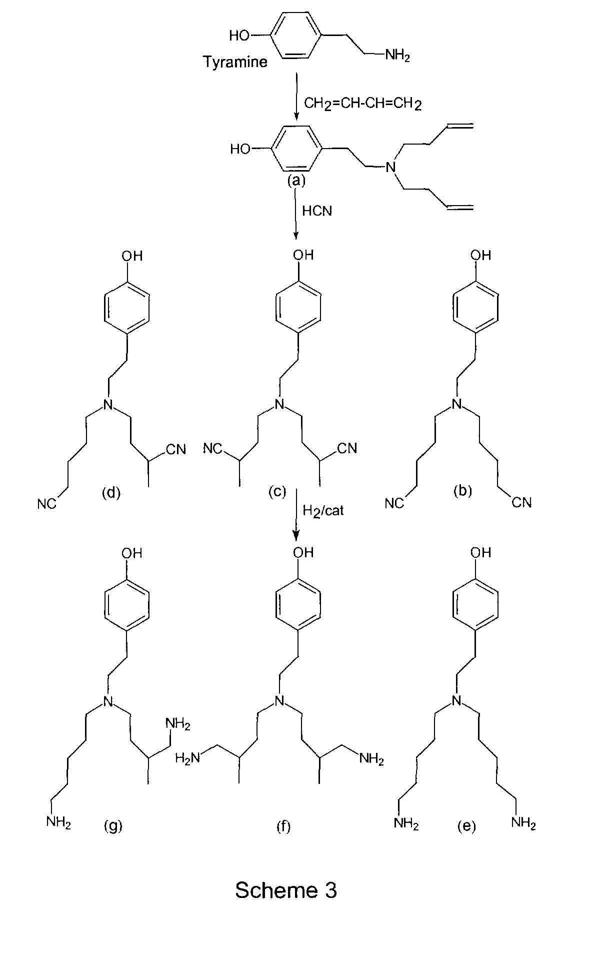

## Claims

1. Compound having the general formula (I), 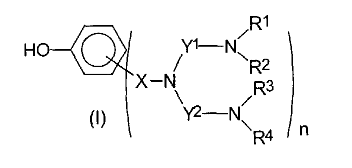 wherein:
n is an integer chosen from 1 to 3,
X, Y¹, Y² represent each independently a bivalent radical, and
R¹, R², R³, R⁴ represent each independently a hydrogen, alkyl or aminoalkyl in which at least one of R¹, R², R³, R⁴ represents hydrogen.

2. Compound according to the general formula (I) of claim 1, wherein Y¹-N(R¹)R² is H.

3. Compound according to any of claims 1 or 2, comprising at least one primary amine.

4. Compound according to any of claims 1 to 3, wherein X is selected from the group consisting of alkyl, alkoxy, aralkyl, thio derivatives optionally substituted by alkyl, aryl, cycloalkyl, halogen, hydroxy, oxy derivatives, thiol, thio derivatives, amino, amino derivatives, amido, amidoxy, nitro, cyano, keto, acyl derivatives, acyloxy derivatives, carboxy, ester, ether, esteroxy, sulfonic acid, sulfonyl derivatives, sulfinyl derivatives, heterocycle, alkenyl or alkynyl, and Y¹, Y² each independently are selected from the group consisting of alkyl, alkoxy, aralkyl, optionally substituted by alkyl, aryl, cycloalkyl, halogen, hydroxy, oxy derivatives, thiol, thio derivatives, amino, amino derivatives, amido, amidoxy, nitro, cyano, keto, acyl derivatives, acyloxy derivatives, carboxy, ester, ether, esteroxy, sulfonic acid, sulfonyl derivatives, sulfinyl derivatives, heterocycle, alkenyl or alkynyl.

5. Compound according to any of claims 1 to 4, wherein X, Y¹, Y² represent each independently a C₁₋₆alkyl.

6. Compound according to any of claims 1 to 5, wherein R¹, R², R³, R⁴ represent each independently a hydrogen or a C₁₋₆ alkyl or C₁₋₆ aminoalkyl.

7. Compound according to any of claims 1 to 6, wherein X, Y¹, Y² each independently are selected from the group consisting of ethyl, propyl, butyl and pentyl.

8. Compound according to any of claims 1 to 7, wherein at least two of R¹, R², R³, R⁴ are hydrogen, preferably at least three and most preferably four of R¹, R², R³, R⁴ are hydrogen.

9. Compound according to any of claims 1 to 8, wherein n is 1.

10. Compound according to any of claims 1 to 9, being para substituted.

11. Compound according to any of claims 1 to 10, having the formula of
N,N-(diaminopropyl)-4-(2-aminoethyl)phenol,
N,N-(diaminopropyl)-4-(2-aminoethyl-1-ol)phenol,
N,N-(diaminopropyl)-4-hydroxyphenylglycine,
N,N-(diaminopropyl)-4-hydroxyphenylglycine methyl ester,
N,N-(diaminopropyl)-tyrosine, or
N,N-(diaminopropyl)-tyrosine methyl ester.

12. Method for the preparation of compound of any of claims 1 to 11, comprising the steps of
(a) reacting an amino phenolic compound of formula (II) with a suitable reagent resulting in an intermediate compound of formula (III), and 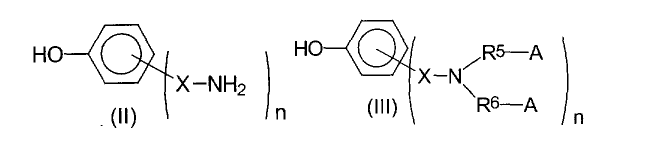
- (b) reducing said compound of formula (III) in order to obtain a compound of formula (I), 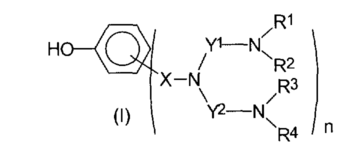
- (c) optionally iterating step (a) and (b) on each compound obtained after step (b),
wherein, R⁵ and R⁶ correspond respectively to Y¹ and Y² with one CH₂ moeity less,A is -C≡N or NO₂ and n is an integer chosen from 1 to 3, X, Y¹, Y² represent each independently a bivalent radical, and R¹, R², R³, R⁴ represent each independently a hydrogen, alkyl or aminoalkyl in which at least one of R¹, R², R³, R⁴ represents hydrogen.

13. Method according to claim 12, wherein R⁵-A is H in compound of formula (III) and Y¹-N(R¹)R² is H in compound of formula (I).

14. Method according to any of claims 12 or 13, wherein the reduction is a catalytic hydrogenation.

15. Method according to any of claims 13 or 13, wherein the suitable reagent is of formula (IVa) or (IVb), 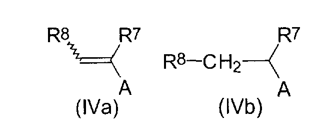 wherein A is -C≡N or NO₂, R⁷ is H or CH₃, R⁸ is H, CH₃, a hydrocarbon radical having 2-18 carbon atoms and containing at least one double bond optionally conjugated with the double bond of formula (IVa) or -R⁹-A wherein R⁹ is a C₁₋₆ alkyl or C₂₋₆ alkenyl radical containing at least one double bond optionally conjugated with the double bond of formula (IVa) and A has the same meaning as that defined above.

16. Method according to claim 14, wherein said suitable reagent is methacrylonitrile or acrylonitrile

17. Intermediates obtainable by the method of any of claims 12 to 16.

18. Intermediates according to claim 17 having the formula of
N,N-(dicyanopropyl)-4-(2-aminoethyl)phenol,
N,N-(dicyanopropyl)-4-(2-aminoethyl-1-ol)phenol,
N,N-(dicyanopropyl)-4-hydroxyphenylglycine,
N,N-(dicyanopropyl)-4-hydroxyphenylglycine methyl ester,
N,N-(dicyanopropyl)-tyrosine, or
N,N-(dicyanopropyl)-tyrosine methyl ester.

19. Use of a compound of any of claims 1 to 11 in an epoxy coating.

20. Use according to claim 19 as a hardener.

21. Use according to claims 19 as an accelerator.

22. A coating composition comprising an epoxy resin, and a compound of any of claims 1 to 11, wherein said compound is preferably in an amount ranging from 0 to 70 % by weight, when used as hardener, and in an amount ranging from 0 to 30 % by weight when used as accelerator.
